# EUROPÄISCHE PATENTSCHRIFT

(11) **EP 1 781 663 B1**
(45) Veröffentlichungstag und Bekanntmachung des Hinweises auf die Patenterteilung: **20.11.2013**
(21) Anmeldenummer: 05777343.4
(22) Anmeldetag: 16.08.2005
(51) Int. Cl.: C07D 487/04, C07D 487/10, C07D 498/10, A61K 31/407, A61K 31/424, A61P 3/04, A61P 3/10

(54) **ARYLSUBSTITUIERTE POLYCYCLICHE AMINE, VERFAHREN ZU IHRER HERSTELLUNG UND IHRE VERWENDUNG ALS ARZNEIMITTEL**
ARYL-SUBSTITUTED POLYCYCLIC AMINES, METHOD FOR THE PRODUCTION THEREOF, AND USE THEREOF AS A MEDICAMENT
AMINES POLYCYCLIQUES SUBSTITUES PAR ARYLE, PROCEDE DE PRODUCTION ASSOCIE ET LEUR UTILISATION EN TANT QUE MEDICAMENTS

(30) Priorität: 16.08.2004 DE 102004039789
(43) Veröffentlichungstag der Anmeldung: 09.05.2007
(73) Patentinhaber: Sanofi-Aventis Deutschland GmbH, 65929 Frankfurt am Main (DE)
(72) Erfinder: SCHWINK, Lothar, 35260 stadtallendorf (DE); STENGELIN, Siegfried, 65817 Eppstein (DE); GOSSEL, Matthias, 65719 Hofheim (DE); HESSLER, Gerhard, 65719 Hofheim (DE); LENNIG, Petra, 55131 Mainz (DE)
(86) Internationale Anmeldenummer: PCT/EP2005/008889
(87) Internationale Veröffentlichungsnummer: WO 2006/018280

(56) Entgegenhaltungen:
- EP-A- 1 249 233
- WO-A-00/53591
- WO-A-01/57037
- WO-A-03/015769
- WO-A-2005/002577

## Beschreibung

Die Erfindung betrifft arylsubstituierte polycyclische Amine, insbesondere bicyclische Amine, sowie deren physiologisch verträgliche Salze und physiologisch funktionelle Derivate.

Es sind bereits den hier beschriebenen arylsubstituierten polycyclischen Aminen in ihrer Gesamtstruktur ähnliche Verbindungen mit pharmakologischer Wirkung im Stand der Technik beschrieben. So beschreibt z. B. W02000053591 Ureido substituierte Azabicyclen mit antiviraler Wirkung. In W02004024702 werden unter anderem Amidoalkylaryl substituierte Azabicyclen mit MCH-antagonistischer Wirkung zur Behandlung von Obesitas beansprucht. In W02003015769 werden strukturähnliche aminoalkyl substituierte aromatische Bicyclen mit MCH-antagonistischer Wirkung zur Behandlung von Obesitas beansprucht.

Verbindungen mit MCH-antagonistischer Wirkung zur Behandlung der Obesitas sind im Stand der Technik beschrieben (Beispiele: W02001021577, W02003035624, W02002089729, W02002006245, WO2002002744, W02002057233, WO2003045313, W02003097047, W02002010146, WO 2003087044).

Der Erfindung lag die Aufgabe zugrunde, Verbindungen zur Verfügung zu stellen, die eine Gewichtsreduktion bei Säugetieren bewirken und die zur Prävention und Behandlung von Obesitas und Diabetes sowie deren vielfältigen Folgeerkrankungen geeignet sind.

Überraschenderweise wurde eine Serie von Verbindungen gefunden, die die Aktivität von MCH-Rezeptoren modulieren. Insbesondere zeichnen sich die Verbindungen durch einen Antagonismus des MCH1R aus.

Die Erfindung betrifft daher Verbindungen der Formel I, 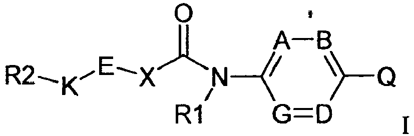 worin bedeuten
- A, B, D, G: unabhängig voneinander N, C(R3) oder die Gruppen A und B oder D und G sind jeweils C(R3) und bilden gemeinsam eine ortho-Phenyleneinheit, so dass sich insgesamt ein 1,4-bisubstituiertes Naphthalinsystem ergibt; bevorzugt unabhängig voneinander N oder C(R3), wobei die Gesamtzahl der Stickstoffatome in dem Ring 0 - 2, bevorzugt 0 oder 1 beträgt, besonders bevorzugt C(R3);
- R3: H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀-C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, Cl, (C₁-C₆)-Alkyl;
- R4, R5, R6, R7, R8: unabhängig voneinander H, (C₁-C₈)-Alkyl;
oder
- R4 und R5, R6 und R7: bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
- R9, R12: unabhängig voneinander H, (C₁-C₈)-Alkyl;
- R13, R14: H;
- R15: H, (C₁-C₆)-Alkyl;
- x: 0, 1, 2, bevorzugt 0, 1, besonders bevorzugt 1;
- R1: H, (C₁-C₈)-Alkyl;
- X: N(R16), eine Bindung, (R17)C=C(R18), C≡C, CH₂-CH₂, YCH₂, CH₂Y, bevorzugt N(R16), eine Bindung;
- Y: O, S, N(R21);
- R16, R17, R18: unabhängig voneinander H, (C₁-C₈)-Alkyl; bevorzugt H;
- R21: H, (C₁-C₈)-Alkyl;
- E: 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, O-(C₃-C₈)-Cycloalkyl, (C₃-C₈)-Cycloalkenyl, (C₂-C₆)-Alkinyl, (C₀-C₈)-Alkylen-aryl, O-(C₀-C₈)-Alkylen-aryl, S-Aryl, N(R22)(R23), SO₂-CH₃, N(R26)CO(R27), N(R28)SO₂(R29), CO(R30) tragen und mono- oder bicyclisch sein kann; bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, NO₂, CN, OCF₃, O-(C₁-C₆)-Alkyl, S-(C₁-C₆)-Alkyl, (C₁-C₆)-Alkyl, (C₂-C₆)-Alkenyl, O-(C₀-C₈)-Alkylen-aryl, S-Aryl, N(R22)(R23), SO₂-CH₃, N(R26)CO(R27), CO(R30) tragen und mono- oder bicyclisch sein kann; besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl, tragen kann z.B. ist E ausgewählt aus der Gruppe bestehend aus 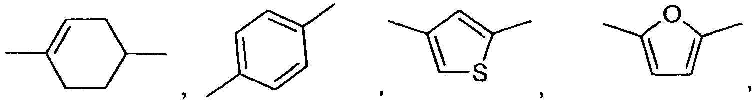 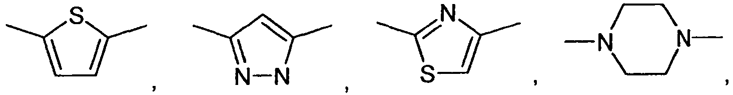 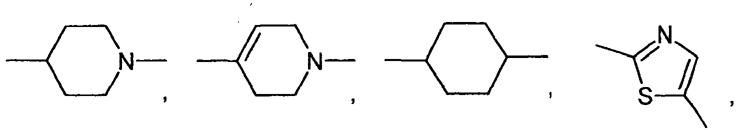 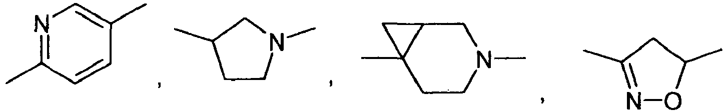 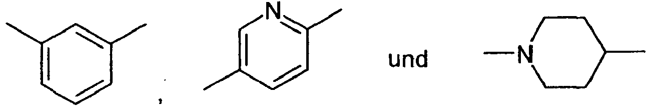
die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl, tragen können;
bevorzugt 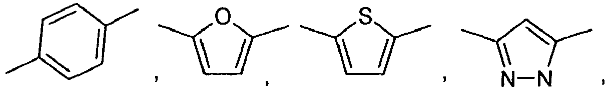 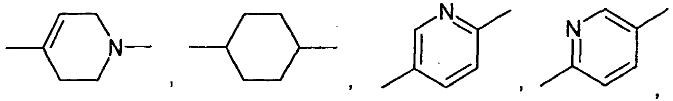 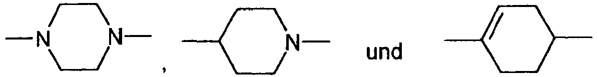 die optional die vorstehend genannten Substituenten tragen können;
- R22, R23, R24, R25, R26, R28: unabhängig voneinander H, (C₁-C₈)-Alkyl;
oder
- R22 und R23, R24 und R25: unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R27, R29, R30: unabhängig voneinander H, (C₁-C₈)-Alkyl;
- K: eine Bindung, O, OCH₂, CH₂O, S, SO, SO₂, N(R35), N(R36)CO, CON(R37), (C(R38)(R39))ᵥ, CO, (R31)C=C(R32), C=C, SCH₂, SO₂CH₂, besonders bevorzugt OCH₂, CH₂O, N(R36)CO, CON(R37), (C(R38)(R39))₂, (R31)C=C(R32), C=C, SCH₂, SO₂CH₂, ganz besonders bevorzugt OCH₂, CH₂O, CON(R37), C≡C, SCH₂;
- v: 2;
- R31, R32, R35, R36, R37, R38, R39: unabhängig voneinander H, (C₁-C₈)-Alkyl;
- R2: (C₁-C₈)-Alkyl, (C₁-C₄)-Alkoxy-(C₁-C₄)-alkyl, ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
- R41, R42, R43, R45, R46, R47: unabhängig voneinander H, (C₁-C₈)-Alkyl;
oder
- R42 und R43, R46 und R47: bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
- Q: bi-, tri- oder spirocyclisches gesättigtes oder teilweise ungesättigtes Ringgerüst mit einem Stickstoffatom und 0-3 weiteren Heteroatomen ausgewählt aus der Gruppe N, O und S, wobei die Ringe des Gerüsts spiro-verknüpft, kondensiert oder verbrückt sein können, und wobei das Ringsystem substituiert sein kann mit einem oder mehreren der folgenden Substituenten: F, OH, CF₃, CN, OCF₃, Oxo, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
- R51: H, (C₁-C₈)-Alkyl;
- R52, R53: unabhängig voneinander H, (C₁-C₈)-Alkyl, OH, (C₃-C₈)-Cycloalkyl, (C₁-C₄)-Alkoxy-(C₁-C₄)-alkyl;
- o, p: unabhängig voneinander 0, 1, 2, 3, 4, 5, 6;
- R54, R55: unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
- R56, R57, R58, R59, R62, R63: unabhängig voneinander H, (C₁-C₈)-Alkyl;
oder
- R56 und R57: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R60, R61: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
- R64, R65, R66, R68, R69, R71, R72, R73,:
- R74, R75, R76, R77, R78: unabhängig voneinander H, (C₁-C₈)-Alkyl;
oder
- R76 und R77: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R67, R70: unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
- R79, R80, R81, R82, R83, R84, R85, R86: unabhängig voneinander H, (C₁-C₈)-Alkyl;
oder
- R79 und R80, R83 und R84: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.
deren N-Oxide sowie deren physiologisch verträgliche Salze.

Die Erfindung bezieht sich auf Verbindungen der Formel I, in Form ihrer Racemate, enantiomerenangereicherten Mischungen und reinen Enantiomere sowie auf ihre Diastereomere und Mischungen davon.

Die Verbindungen der Formel I zeichnen sich dadurch aus, dass sie gegenüber strukturell ähnlichen Verbindungen eine verbesserte metabolische Stabilität bei gleichzeitiger hoher Aktivität aufweisen.

Die Alkyl-, Alkenyl- und Alkinylreste in den Substituenten R1, R2, R3, R4, R5, R6, R7, R8, R9, R10, R11, R12, R13, R14, R15, R16, R17, R18, R19, R20, R21, R22, R23, R24, R25, R26, R27, R28, R29, R30, R31, R32, R33, R34, R35, R36, R37, R38, R39, R40, R41, R42, R43, R44, R45, R46, R47, R51, R52, R53, R54, R55, R56, R57, R58, R59, R60, R61, R62, R63, R64, R65, R66, R67, R68, R69, R70, R71, R72, R73, R74, R75, R76, R77, R78, R79, R80, R81, R82, R83, R84, R85, R86, R87, R88, R89 und R90 können sowohl geradkettig, verzweigt und/oder optional substituiert sein mit Substituenten wie Aryl, Heteroaryl, Alkoxy oder Halogen. Dies trifft auch zu, sofern die Alkyl-, Alkenyl- und Alkinylreste Teil einer anderen Gruppe sind, z.B. Teil einer Alkoxygruppe (wie (C₁-C₄)-Alkoxy-(C₁-C₄)-alkyl)). Geeignete Halogene sind Fluor, Chlor, Brom und Iod, bevorzugt Fluor, Chlor und Brom, besonders bevorzugt Fluor.

Beispiele für Alkylgruppen sind: Methyl, Ethyl, Propyl, Butyl, Pentyl, Hexyl, Heptyl und Octyl. Dabei sind sowohl die n-Isomere dieser Reste als auch verzweigte Isomere wie Isopropyl, Isobutyl, Isopentyl, sec-Butyl, tert-Butyl, Neopentyl, 3,3-Dimethylbutyl usw. mit umfasst. Sofern nicht anders beschrieben, beinhaltet der Begriff Alkyl darüber hinaus auch Alkylreste, die unsubstituiert oder gegebenenfalls mit einem oder mehreren weiteren Resten substituiert sind, beispielsweise mit 1, 2, 3 oder 4 identischen oder unterschiedlichen Resten, wie Aryl, Heteroaryl, (C₁-C₄)-Alkoxy oder Halogen. Dabei können die zusätzlichen Substituenten in jeder beliebigen Position des Alkylrestes auftreten. Bevorzugt sind die Alkylreste -soweit nicht anders definiert- unsubstituiert.

Unter Cycloalkyl ist im Sinne der vorliegenden Anmeldung Cycloalkyl sowie Cycloalkylalkyl- (Alkyl, das wiederum mit Cycloalkyl substituiert ist) zu verstehen, wobei Cycloalkyl mindestens 3 Kohlenstoffatome aufweist. Beispiele für Cycloalkylreste sind: Cyclopropyl, Cyclobutyl, Cyclopentyl, Cyclohexyl, Cycloheptyl, Cyclooctyl, Cyclononyl und Cyclodecyl. Gegebenenfalls kann es sich auch um polycyclische Ringsysteme handeln, wie Decalinyl, Norbornanyl, Bornanyl oder Adamantanyl. Die Cycloalkylreste können unsubstituiert oder gegebenenfalls mit einem oder mehreren weiteren Resten substituiert sein, wie vorstehend bei den Alkylresten beispielhaft aufgeführt. Bevorzugt sind die Cycloalkylreste -soweit nicht anders definiert- unsubstituiert.

Beispiele für Alkenyl- und Alkinylgruppen sind: Vinyl, 1-Propenyl, 2-Propenyl (Allyl), 2-Butenyl, 2-Methyl-2-propenyl, 3-Methyl-2-butenyl, Ethinyl, 2-Propinyl (Propargyl), 2-Butinyl oder 3-Butinyl.

Unter Cycloalkenyl sind im Sinne der vorliegenden Anmeldung Cycloalkenylreste sowie Cycloalkenyl-Alkylreste (Alkyl, das mit Cycloalkenyl substituiert ist) zu verstehen, die mindestens drei Kohlenstoffatome enthalten. Beispiele für Cycloalkenyl sind: Cyclopentenyl, Cyclohexenyl, Cycloheptenyl und Cyclooctenyl.

Die Alkenylreste und Cycloalkenylreste können ein bis drei konjugierte oder nicht konjugierte Doppelbindungen (also auch Alk-dienyl- sowie Alk-trienylreste), vorzugsweise eine Doppelbindung in einer geraden oder verzweigten Kette aufweisen. Für Alkinylreste gilt das gleiche für die Dreifachbindungen. Die Alkenyl- und Alkinylreste können unsubstituiert oder gegebenenfalls mit einem oder mehreren weiteren Resten substituiert sein, wie vorstehend bei den Alkylresten beispielhaft aufgeführt. Bevorzugt sind die Alkenyl- und Alkinylreste -soweit nicht anders definiert- unsubstituiert.

Als Aryl werden in der vorliegenden Erfindung Reste bezeichnet, die von monocyclischen oder bicyclischen Aromaten abgeleitet sind, die keine Ringheteroatome enthalten. Sofern es sich nicht um monocyclische Systeme handelt, ist bei der Bezeichnung Aryl für den zweiten Ring auch die gesättigte Form (Perhydroform) oder die teilweise ungesättigte Form (beispielsweise die Dihydroform oder Tetrahydroform), sofern die jeweiligen Formen bekannt und stabil sind, möglich. Die Bezeichnung Aryl umfasst in der vorliegenden Erfindung beispielsweise auch bicyclische Reste, in denen sowohl beide Ringe aromatisch sind als auch bicyclische Reste, in denen nur ein Ring aromatisch ist. Beispiele für Aryl sind: Phenyl, Naphthyl, Indanyl, 1,2-Dihydronaphthenyl, 1,4-Dihydronaphthenyl, Indenyl oder 1,2,3,4-Tetrahydronaphthyl. Bevorzugt sind die Arylreste-soweit nicht anders definiert- unsubstituiert. Besonders bevorzugt ist Aryl Phenyl oder Naphthyl.

Unter Heteroarylresten sind Reste zu verstehen, die von monocyclischen oder bicyclischen Aromaten abgeleitet sind, die Ringheteroatome, bevorzugt N, O oder S, enthalten. Im Übrigen gilt für die Heteroarylreste das bezüglich der Arylreste Aufgeführte.

Unter einem "Tricyclus" werden Strukturen mit 3 Ringen verstanden, die durch mehr als eine Bindung miteinander verbunden sind. Beispiele solcher Systeme sind kondensierte Systeme mit 3 Ringen und Spirocyclen mit ankondensiertem Ringsystem.

Unter die bivalente carbo- oder heterocyclische Ringstruktur E fallen auch Strukturen, die über ein und dasselbe Atom mit den beiden benachbarten Gruppen K und X verknüpft sind.

Unter einer polycyclischen Gruppe ist im Sinne der vorliegenden Anmeldung eine Gruppe zu verstehen, die von Spiranen, kondensierten Ringsystemen oder Brücken-Ringsystemen abgeleitet ist. Die Spirane zeichnen sich dadurch aus, dass zwei Ringe nur ein Kohlenstoffatom gemeinsam aufweisen und die Ringebenen der beiden Ringe senkrecht aufeinander stehen. Bei den kondensierten Ringsystemen sind zwei Ringe so miteinander verknüpft, dass sie zwei Atome gemeinsam aufweisen. Bei dieser Art der Verknüpfung handelt es sich um eine "*ortho*-Kondensation". Bei den Brücken-Ringsystemen handelt es sich um Ringsysteme, die eine Brücke aus Kohlenstoff- und/oder Heteroatomen zwischen zwei nicht benachbarten Atomen eines Rings aufweisen.

Unter einem "chemisch sinnvollen Rest" ist im Sinne der vorliegenden Erfindung ein Rest zu verstehen, der bei Raumtemperatur und Normaldruck stabil ist. Bevorzugt sind im Sinne der vorliegenden Erfindung unter einem "chemisch sinnvollen Rest" in den Definitionen der Gruppen X und K in den Verbindungen der Formel (I) Gruppen der Formel (CR19R20)_{y} (in der Definition von X) bzw. (CR33R34)_{z} (in der Definition von K) zu verstehen, die keine Heteroatom-Heteroatom-Bindungen zwischen den einzelnen Gruppen (CR19R20) bzw. (CR33R34) aufweisen.

Die Verbindungen der Formel I können ein oder mehrere Assymmetriezentren enthalten. Daher können die Verbindungen der Formel I in Form ihrer Racemate, Enantiomeren angereicherten Mischungen, reinen Enantiomere, Diastereomere und Diastereomer-Mischungen vorliegen. Die vorliegende Erfindung umfasst alle diese isomeren Formen der Verbindungen der Formel I. Diese Isomeren Formen können, wenn auch zum Teil nicht expressis verbis beschrieben, nach bekannnten Methoden erhalten werden.

Pharmazeutisch verträgliche Salze sind aufgrund ihrer höheren Wasserlöslichkeit gegenüber den Ausgangs- bzw. Basisverbindungen besonders geeignet für medizinische Anwendungen. Diese Salze müssen ein pharmazeutisch verträgliches Anion oder Kation aufweisen. Geeignete pharmazeutisch verträgliche Säureadditionssalze der erfindungsgemäßen Verbindungen sind Salze anorganischer Säuren, wie Salzsäure, Bromwasserstoff-, Phosphor-, Metaphosphor-, Salpeter- und Schwefelsäure sowie organischer Säuren, wie z.B. Essigsäure, Benzolsulfon-, Benzoe-, Zitronen-, Ethansulfon-, Fumar-, Glucon-, Glykol-, Isethion-, Milch-, Lactobion-, Malein-, Äpfel-, Methansulfon-, Bernstein-, p-Toluolsulfon- und Weinsäure. Geeignete pharmazeutisch verträgliche basische Salze sind Ammoniumsalze, Alkalimetallsalze (wie Natrium- und Kaliumsalze) und Erdalkalisalze (wie Magnesium- und Calciumsalze) und Salze von Trometamol (2-Amino-2-hydroxymethyl-1,3-propandiol), Diethanolamin, Lysin oder Ethylendiamin.

Salze mit einem nicht pharmazeutisch verträglichen Anion, wie zum Beispiel Trifluoracetat, gehören ebenfalls in den Rahmen der Erfindung als nützliche Zwischenprodukte für die Herstellung oder Reinigung pharmazeutisch verträglicher Salze und/oder für die Verwendung in nicht-therapeutischen, zum Beispiel in-vitro-Anwendungen.

Der hier verwendete Begriff "physiologisch funktionelles Derivat" bezeichnet jedes physiologisch verträgliche Derivat einer erfindungsgemäßen Verbindung der Formel I, z.B. einen Ester, der bei Verabreichung an einen Säuger, wie z.B. den Menschen, in der Lage ist, (direkt oder indirekt) eine Verbindung der Formel I oder einen aktiven Metaboliten hiervon zu bilden.

Zu den physiologisch funktionellen Derivaten zählen auch Prodrugs der erfindungsgemäßen Verbindungen, wie zum Beispiel in H. Okada et al., Chem. Pharm. Bull. 1994, 42, 57-61 beschrieben. Solche Prodrugs können in vivo zu einer erfindungsgemäßen Verbindung metabolisiert werden. Diese Prodrugs können selbst wirksam sein oder nicht.

Die erfindungsgemäßen Verbindungen können auch in verschiedenen polymorphen Formen vorliegen, z.B. als amorphe und kristalline polymorphe Formen. Alle polymorphen Formen der erfindungsgemäßen Verbindungen gehören in den Rahmen der Erfindung und sind ein weiterer Aspekt der Erfindung.

Nachfolgend beziehen sich alle Verweise auf "Verbindung(en) gemäß Formel I" auf Verbindung(en) der Formel I, wie vorstehend beschrieben, sowie ihre Salze, Solvate und physiologisch funktionelle Derivate wie hierin beschrieben.

Können Reste oder Substituenten mehrfach in den Verbindungen der Formel I auftreten, so können sie alle unabhängig voneinander die angegebenen Bedeutungen haben und gleich oder verschieden sein.

Bevorzugt weisen die Symbole in Verbindung I die folgenden Bedeutungen auf:
- A, B, D, G: unabhängig voneinander N oder C(R3) bedeuten und die Gesamtzahl der Stickstoffatome in diesem Ring 0-2, bevorzugt 0 oder 1, besonders bevorzugt 0 beträgt.

Die Verknüpfung zwischen der Gruppe 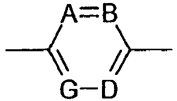 und Q erfolgt bevorzugt über ein Stickstoffatom, das innerhalb des Ringgerüstes Q vorliegt.

In einer Ausführungsform der vorliegenden Erfindung bedeutet Q in den Verbindungen der Formel I
ein bi-, tri- oder spirocyclisches gesättigtes oder teilweise ungesättigtes Ringgerüst mit einem Stickstoffatom und 0-3 weiteren Heteroatomen ausgewählt aus der Gruppe N, O und S, wobei die Ringe des Gerüsts spiro-verknüpft, kondensiert oder verbrückt sein können, und wobei das Ringsystem substituiert sein kann mit einem oder mehreren der folgenden Substituenten F, OH, CF₃, CN, OCF₃, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)p-R55;
worin bedeuten
- R51: H, (C₁-C₈)-Alkyl;
- R52, R53: unabhängig voneinander H, (C₁-C₈)-Alkyl, OH, (C₃-C₈)-Cycloalkyl, (C₁-C₄)-Alkoxy-(C₁-C₄)alkyl;
- o, p: unabhängig voneinander 0, 1, 2, 3, 4, 5, 6;
- R54, R55: unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten F, Cl, Br, CF₃, (C₁-C₈)-Alkyl, O-(C₁-C₈)-Alkyl, CO(R63), OH;
- R56, R57, R58, R59, R62, R63: unabhängig voneinander H, (C₁-C₈)-Alkyl;
oder
- R56 und R57: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R60, R61: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
- R64, R65, R66, R67, R68, R69,:
- R71, R72, R73, R74, R75, R76, R77, R78: unabhängig voneinander H, (C₁-C₈)-Alkyl;
oder
- R76 und R77: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R67, R70: unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R86), Oxo, OH;
- R79, R80, R81, R82, R83, R84, R85, R86: unabhängig voneinander H, (C₁-C₈)-Alkyl;
oder
- R79 und R80, R83 und R84: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

In einer weiteren Ausführungsform der vorliegenden Erfindung bedeutet Q in den Verbindungen der Formel I ein bi-, tri- oder spirocyclisches gesättigtes Ringgerüst mit einem Stickstoffatom und 0-3 weiteren Heteroatomen ausgewählt aus der Gruppe N, O und S, wobei die Ringe des Gerüsts spiro-verknüpft, kondensiert oder verbrückt sein können, und wobei das Ringsystem substituiert sein kann mit einem oder mehreren der folgenden Substituenten: F, OH, CF₃, CN, OCF₃, Oxo, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
worin bedeuten
- R51: H, (C₁-C₈)-Alkyl;
- R52, R53: unabhängig voneinander H, (C₁-C₈)-Alkyl, OH, (C₃-C₈)-Cycloalkyl, (C₁-C₄)-Alkoxy-(C₁-C₄)-alkyl;
- o, p: unabhängig voneinander 0, 1, 2, 3, 4, 5, 6;
- R54, R55: unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R63), Oxo, OH;
- R56, R57, R58, R59, R62, R63: unabhängig voneinander H, (C₁-C₈)-Alkyl;
oder
- R56 und R57: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R60, R61: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)q-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
- R64, R65, R66, R68, R69, R71,:
- R72, R73, R74, R75, R76, R77, R78: unabhängig voneinander H, (C₁-C₈)-Alkyl;
oder
- R76 und R77: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R67, R70: unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
- R79, R80, R81, R82, R83, R84, R85, R86: unabhängig voneinander H, (C₁-C₈)-Alkyl;
oder
- R79 und R80, R83 und R84: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

Besonders bevorzugt weist die Gruppe Q in den Verbindungen der Formel I die folgenden Bedeutungen auf: 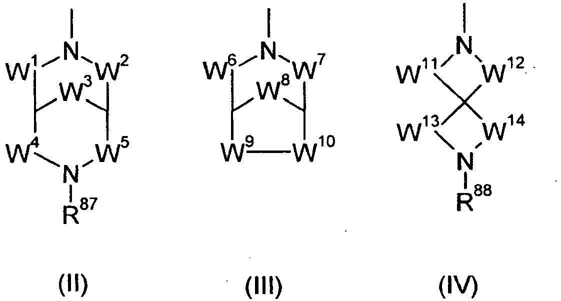 worin bedeuten
W1, W2, W3, W4, W5, W6, W7, W8, W9, W10, W11, W12, W13, W14 unabhängig voneinander eine Bindung, C=C, 1 bis 4-gliedrige Alkylen- oder Alkylidenkette, in der 0-1 Kohlenstoffatome außerhalb einer in der Alkylidenkette vorhandenen Doppelbindung durch ein Element aus der Gruppe N(R90), O und S ersetzt sein können;
wobei die Kohlenstoffatome in den Gruppen der Formeln (II), (III) und (IV) substituiert sein können mit H, F, OH, Oxo, (C₁-C₆)-Alkyl, O-(C₁-C₆)-Alkyl, (CR52R53)ₒR54, bevorzugt H, (CR52R53)ₒR54;
- R87, R88, R90: H, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
- R51: H, (C₁-C₈)-Alkyl;
- R52, R53: unabhängig voneinander H, (C₁-C₈)-Alkyl, OH, (C₃-C₈)-Cycloalkyl, (C₁-C₄)-Alkoxy-(C₁-C₄)-alkyl;
- o, p: unabhängig voneinander 0, 1, 2, 3, 4, 5, 6;
- R54, R55: unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
- R56, R57, R58, R59, R62, R63: unabhängig voneinander H, (C₁-C₈)-Alkyl;
oder
- R56 und R57: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R60, R61: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)q-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
- R64, R65, R66, R68, R69,:
- R71, R72, R73, R74, R75, R76, R77, R78: unabhängig voneinander H, (C₁-C₈)-Alkyl;
oder
- R76 und R77: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1, 2, 3, 4, 5, 6;
- R67, R70: unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
- R79, R80, R81, R82, R83, R84, R85, R86: unabhängig voneinander H, (C₁-C₈)-Alkyl;
oder
- R79 und R80, R83 und R84: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

In einer bevorzugten Ausführungsform bedeuten W3 in Formel II und W8 in Formel III jeweils eine Bindung.

Des Weiteren bevorzugt ist Q ein Rest der Formel IV, worin mindestens einer der beiden Ringe einen 5-gliedrigen Ring darstellt.

Ganz besonders bevorzugt sind Verbindungen der Formel I, worin Q die folgenden Bedeutungen aufweist: 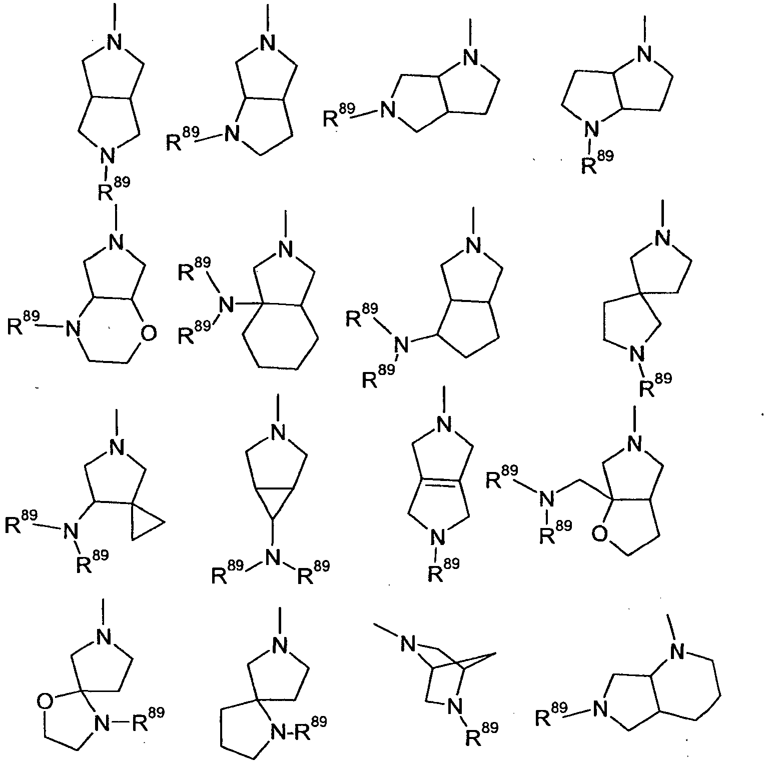 worin bedeuten:
- R89, in der Gruppierung N-R89:: H, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55; bevorzugt H, (C₁-C₆)-Alkyl;
- R51: H, (C₁-C₈)-Alkyl;
- R52, R53: unabhängig voneinander H, (C₁-C₈)-Alkyl, OH, (C₃-C₈)-Cycloalkyl, (C₁-C₄)-Alkoxy-(C₁-C₄)-alkyl;
- o, p: unabhängig voneinander 0, 1, 2, 3, 4, 5, 6;
- R54, R55: unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
- R56, R57, R58, R59, R62, R63: unabhängig voneinander H, (C₁-C₈)-Alkyl;
oder
- R56 und R57: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R60, R61: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
- R64, R65, R66, R68, R69,:
- R71, R72, R73, R74, R75, R76, R77, R78: unabhängig voneinander H, (C₁-C₈)-Alkyl;
oder
- R76 und R77: bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- q, r: unabhängig voneinander 0, 1,2, 3, 4, 5, 6;
- R67, R70: unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R86), Oxo, OH;
- R79, R80, R81, R82, R83, R84, R85, R86: unabhängig voneinander H, (C₁-C₈)-Alkyl;
oder
- R79 und R80, R83 und R84: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R89 in der Gruppierung N(R89)₂:: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)q-R67, CO(CR68R69)ᵣ-R70; oder die beiden Reste R89 bilden zusammen mit dem Stickstoffatom, an das sie gebunden sind, einen 4 bis 10-gliedrigen mono-, bi- oder spirocyclischen Ring, welcher außer dem dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me; bevorzugt H, (C₁-C₆)-Alkyl, oder die beiden Reste R89 bilden zusammen mit dem Stickstoffatom, an das sie gebunden sind, einen 5 bis 6-gliedrigen monocyclischen Ring, welcher außer dem Stickstoffatom 0 bis 1 zusätzliches Heteroatom ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me.

Weiter bevorzugt weist die Gruppe Q die folgenden Bedeutungen auf: 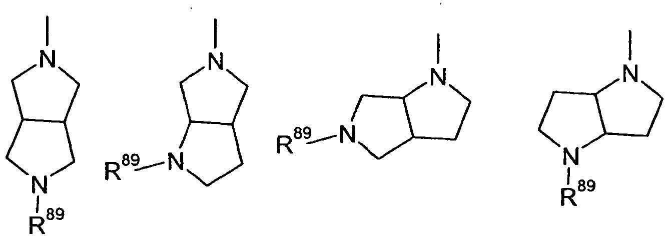 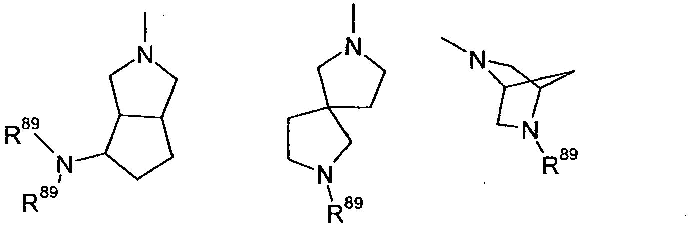 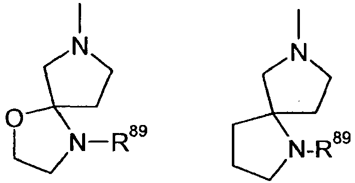 worin R89 die vorstehend genannten Bedeutungen aufweist.

Insbesondere bevorzugt weist die Gruppe Q die folgenden Bedeutungen auf: 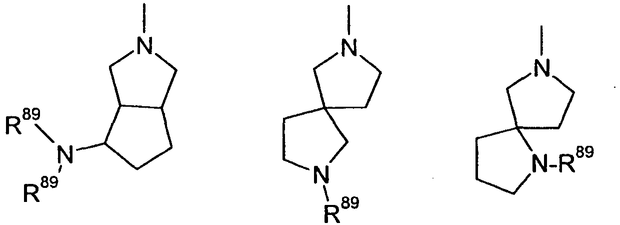 worin R89 die vorstehend genannten Bedeutungen aufweist.

Ganz besonders bevorzugt ist Q 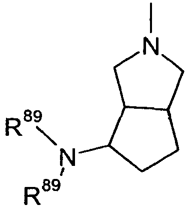 worin R89 die vorstehend genannten Bedeutungen aufweist.

Des Weiteren betrifft die vorliegende Erfindung Verbindungen der Formel I worin
- A, B, D, G: unabhängig voneinander N oder C(R3) bedeuten und die Gesamtzahl der Stickstoffatome in diesem Ring 0-2, bevorzugt 0 oder 1, besonders bevorzugt 0, beträgt;
wobei die weiteren Symbole in Formel I bereits vorstehend definiert wurden.

In einer bevorzugten Ausführungsform betrifft die vorliegende Anmeldung Verbindungen der Formel I worin bedeuten
- A, B, D, G: C(R3);
- R3: H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀-C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)7(R14))ₓ-O(R15); ganz besonders bevorzugt H, F, Cl, (C₁-C₆)-Alkyl; ganz ganz besonders bevorzugt H, CH₃, F;
- R4, R5, R6, R7, R8: unabhängig voneinander H, (C₁-C₈)-Alkyl;
oder
- R4 und R5, R6 und R7: unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
- R9, R12: unabhängig voneinander H, (C₁-C₈)-Alkyl;
- R13, R14: H;
- R15: H, (C₁-C₆)-Alkyl;
- x: 0, 1, 2, bevorzugt 0, 1, besonders bevorzugt 1.

In einer weiteren bevorzugten Ausführungsform bedeuten A, B, G und D in den Verbindungen der Formel I CH.

R2 ist bevorzugt ausgewählt aus der Gruppe bestehend aus:

(C₁-C₈)-Alkyl, (C₁-C₄)-Alkoxy-(C₁-C₄)-alkyl, ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
worin bedeuten:
- R41, R42, R43, R45, R46, R47: unabhängig voneinander H, (C₁-C₈)-Alkyl;
oder
- R42 und R43, R46 und R47: bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

Bevorzugt ist R2 ausgewählt aus n-Propyl, n-Butyl, iso-Butyl, iso-Pentyl, Cyclopropyl, Cyclobutyl, Cyclopentyl, Cyclohexyl, Cyclohex-(1)-enyl, Phenyl, p-Fluorophenyl, p-Chlorophenyl, p-Bromophenyl, p-Tolyl, p-Methoxyphenyl, p-Trifluoromethylphenyl,

K ist bevorzugt ausgewählt aus der Gruppe bestehend aus:

Bindung, O, CO, OCH₂, CH₂O, N(R36)CO, CON(R37), (C(R38)(R39))₂, (R31)C=C(R32), C=C, SCH₂, SO₂CH₂, besonders bevorzugt OCH₂, CH₂O, N(R36)CO, CON(R37), (C(R38)(R39))₂, (R31)C=C(R32), C≡C, SCH₂, SO₂CH₂, ganz besonders bevorzugt OCH₂, CH₂O, CON(R37), (C(R38)(R39))₂, C=C, SCH₂; wobei
R31, R32, R36, R37, R38, R39
unabhängig voneinander H, (C₁-C₈)-Alkyl bedeuten.
X ist bevorzugt ausgewählt aus der Gruppe bestehend aus Bindung und N(R16) bedeutet, worin R16 H oder (C₁-C₈)-Alkyl bedeutet, besonders bevorzugt Bindung und NH.

Die Gruppe E in den Verbindungen der Formel I ist vorstehend definiert. Gemäß den vorstehenden Definitionen für E kann E z.B. ein fünf- oder sechsgliedriger Ring sein. Handelt es sich bei der Gruppe E um einen fünfgliedrigen Ring, so sind die Gruppen K und X in den Verbindungen der Formel I in einer bevorzugten Ausführungsform in der 1- und 3-Position des fünfgliedrigen Rings angeordnet. Handelt es sich bei der Gruppe E um einen sechsgliedrigen Ring, so sind die Gruppen K und X in einer bevorzugten Ausführungsform in der 1- und 4-Position (d.h. in para-Stellung zueinander) des sechsgliedrigen Rings angeordnet.

E ist besonders bevorzugt ausgewählt aus der Gruppe bestehend aus:

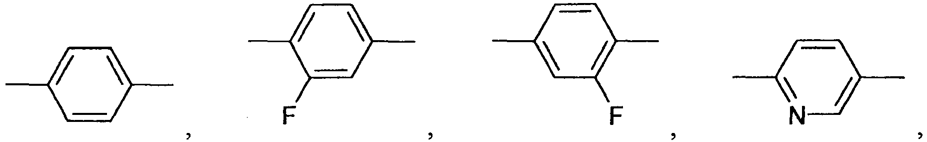

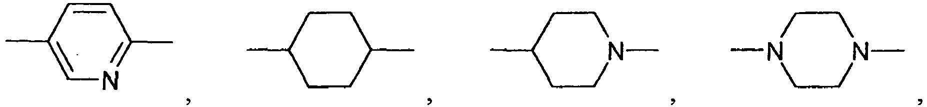

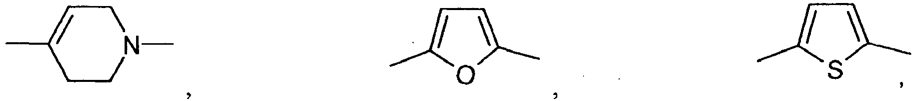

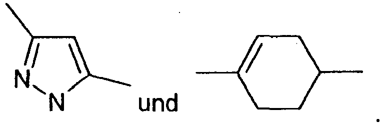

Diese Erfindung bezieht sich weiterhin auf die Verwendung von Verbindungen der Formel I und ihren pharmazeutischen Zusammensetzungen als MCH-Rezeptor-Liganden. Die erfindungsgemäßen MCH-Rezeptor-Liganden eignen sich insbesondere als Modulatoren der Aktivität des MCH1R.

Die Rolle von MCH in der Regulation des Energiehaushalts ist inzwischen gut dokumentiert (Qu, D. et al.; Nature 1996, 380, 243-7; Shimada, M. et al. Nature 1998, 396, 670-4; Chen, Y. et al. Endocrinology 2002, 143, 2469-77; Endocrinology 2003, 144, 4831-40; Übersicht: G. Hervieu, Expert Opin. Ther. Targets 2003, 7, 495-511).

Auch gibt es Hinweise, dass MCH-Antagonisten zentral bedingte Störungen wie z.B. Depressionen günstig beeinflussen können (Borowsky, B. et al.; Nature Medicine 2002, 8, 825-30; Übersicht: G. Hervieu, Expert Opin. Ther. Targets 2003, 7, 495-511).

Besonders geeignet sind solche Verbindungen zur Behandlung und/oder Prävention von

1. Obesitas

## Patentansprüche

1. Verbindungen der Formel I, 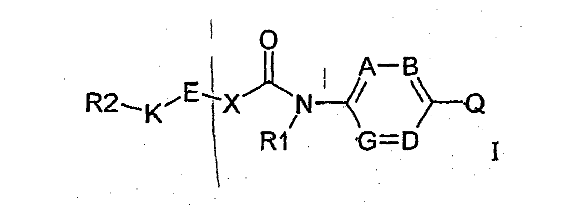 worin bedeuten
A, B, D, G unabhängig voneinander N, C(R3) oder die Gruppen A und B oder D und G sind jeweils C(R3) und bilden gemeinsam eine ortho-Phenyleneinheit, so dass sich insgesamt ein 1,4-bisubstituiertes Naphthalinsystem ergibt; bevorzugt unabhängig voneinander N oder C(R3), wobei die Gesamtzahl der Stickstoffatome in dem Ring 0 - 2, bevorzugt 0 oder 1 beträgt, besonders bevorzugt C(R3);
R3 H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀-C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, Cl, (C₁-C₆)-Alkyl; 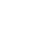
R4, R5, R6, R7, R8 unabhängig voneinander H, (C₁-C₈)-Alkyl;
oder
R4 und R5, R6 und R7 bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
R9, R12 unabhängig voneinander H, (C₁-C₈)-Alkyl;
R13, R14 H;
R15 H, (C₁-C₆)-Alkyl;
x 0, 1, 2, bevorzugt 0, 1, besonders bevorzugt 1;
R1 H, (C₁-C₈)-Alkyl;
X N(R16), eine Bindung, (R17)C=C(R18), C=C, CH₂-CH₂, YCH₂, CH₂Y, bevorzugt N(R16), eine Bindung;
Y O, S, N(R21);
R16, R17, R18 unabhängig voneinander H, (C₁-C₈)-Alkyl; bevorzugt H;
R21 H, (C₁-C₈)-Alkyl;
E 3-8 gliedrige bivalente carbo- oder heterocyclische Ringstruktur mit 0-4 Heteroatomen aus der Gruppe N, O und S, die optional Substituenten aus der Gruppe H, F, Cl, Br, OH, CF₃, NO₂, CN, OCF₃, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₂-C₆)-Alkenyl, O-(C₃-C₈)-Cycloalkyl, (C₃-C₈)-Cycloalkenyl, (C₂-C₆)-Alkinyl; (C₀-C₈)-Alkylen-aryl, O-(C₀-C₈)-Alkylen-aryl, S-Aryl, N(R22)(R23), SO₂-CH₃, N(R26)CO(R27), N(R28)SO₂(R29), CO(R30) tragen und mono- oder bicyclisch sein kann; bevorzugt 5-7 gliedrige bivalente carbo- oder heterocyclische Ringstruktur mit 0-3 Heteroatomen aus der Gruppe N, O und S, die optional Substituenten aus der Gruppe H, F, Cl, Br, OH, CF₃, NO₂, CN, OCF₃, O-(C₁-C₆)-Alkyl, S-(C₁-C₆)-Alkyl, (C₁-C₆)-Alkyl, (C₂-C₆)-Alkenyl, O-(C₀-C₈)-Alkylen-aryl, S-Aryl, N(R22)(R23), SO₂-CH₃, N(R26)CO(R27), CO(R30) tragen und mono- oder bicyclisch sein kann; besonders bevorzugt 5-7 gliedrige bivalente carbo- oder heterocyclische Ringstruktur mit 0-2 Heteroatomen aus der Gruppe N, O und S, die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl, tragen kann z.B. ist E ausgewählt aus der Gruppe bestehend aus 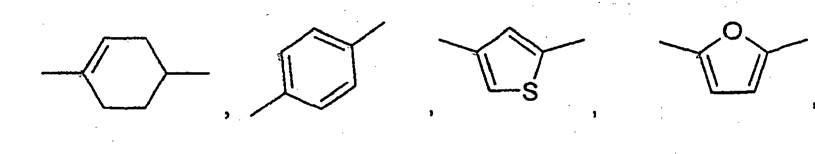 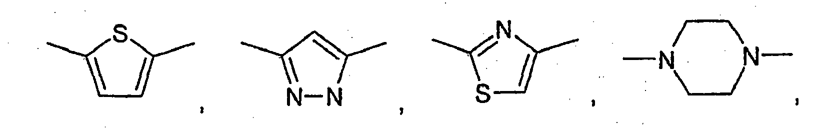 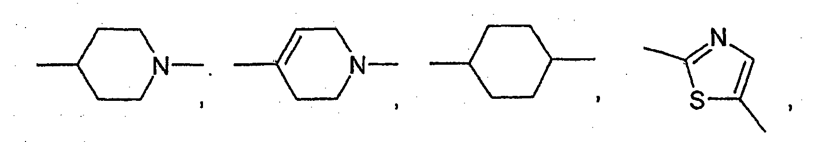 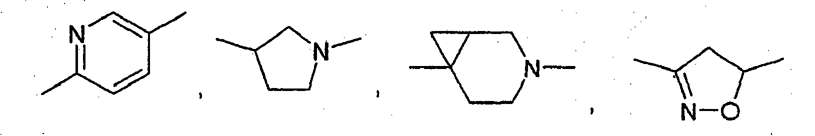 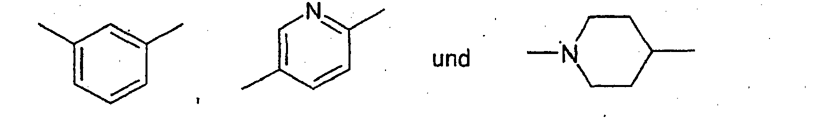
die optional Substituenten aus der Gruppe H, F, Cl, Br, OH, CF₃, NO₂, OCF₃, O-(C₁-C₆)-Alkyl, (C₁-C₆)-Alkyl, (C₂-C₆)-Alkenyl, N(R22)(R23), SO₂-CH₃, CO(R30), bevorzugt H, F, Cl, Br, OH, CF₃, (C₁-C₆)-Alkyl, O-(C₁-C₆)-Alkyl, tragen können; bevorzugt 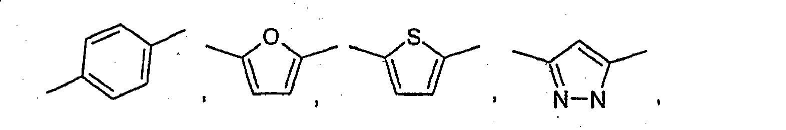 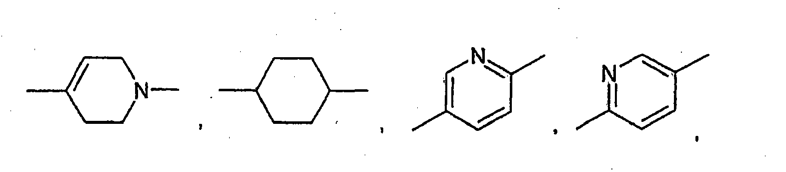 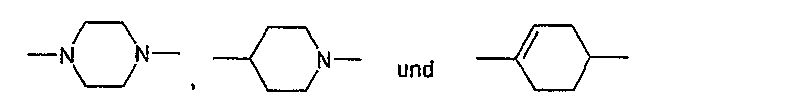 die optional die vorstehend genannten Substituenten tragen können;
R22, R23, R24, R25, R26, R28 unabhängig voneinander H, (C₁-C₈)-Alkyl;
oder
R22 und R23, R24 und R25 unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann; R27, R29, R30 unabhängig voneinander H, (C₁-C₈)-Alkyl;
K eine Bindung, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, besonders bevorzugt OCH₂, CH₂O, N(R36)CO, CON(R37), (C(R38)(R39))₂, (R31)C=C(R32), C=C, SCH₂, SO₂CH₂, ganz besonders bevorzugt OCH₂, CH₂O, CON(R37), C=C, SCH₂;
v 2;
R31, R32, R35, R36, R37, R38, R39 unabhängig voneinander H, (C₁-C₈)-Alkyl;
R2 (C₁-C₈)-Alkyl, (C₁-C₄)-Alkoxy-(C₁-C₄)-alkyl, ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
R41, R42, R43, R45, R46, R47 unabhängig voneinander H, (C₁-C₈)-Alkyl;
oder
R42 und R43, R46 und R47 bilden unabhängig voneinander optional zusammen mit dem Stickstoffatom, an das sie gebunden sind, einen 5-6 gliedrigen Ring, welcher ausser dem Stickstoffatom noch 0-1 weitere Heteroatome aus der Gruppe NH, N-(C₁-C₆)-Alkyl, Sauerstoff und Schwefel beinhalten kann;
Q bi-, tri- oder spirocyclisches gesättigtes oder teilweise ungesättigtes Ringgerüst mit einem Stickstoffatom und 0-3 weiteren Heteroatomen ausgewählt aus der Gruppe N, O und S, wobei die Ringe des Gerüsts spiroverknüpft, kondensiert oder verbrückt sein können, und wobei das Ringsystem substituiert sein kann mit einem oder mehreren der folgenden Substituenten: F, OH, CF₃, CN, OCF₃, Oxo, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
R56, R57, R58, R59, R62, R63 unabhängig voneinander H, (C₁-C₈)-Alkyl;
oder
R56 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N(C₁-C₆)-Alkyl, Sauerstoff und Schwefel beinhalten kann;
R60, R61 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
R64, R65, R66, R68, R69,
R71, R72, R73, R74, R75, R76, R77, R78 unabhängig voneinander H, (C₁-C₈)-Alkyl;
oder
R76 und R77 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann. deren N-Oxide sowie deren physiologisch verträgliche Salze.

2. Verbindungen nach Anspruch 1,
worin
Q die folgenden Bedeutungen aufweist: 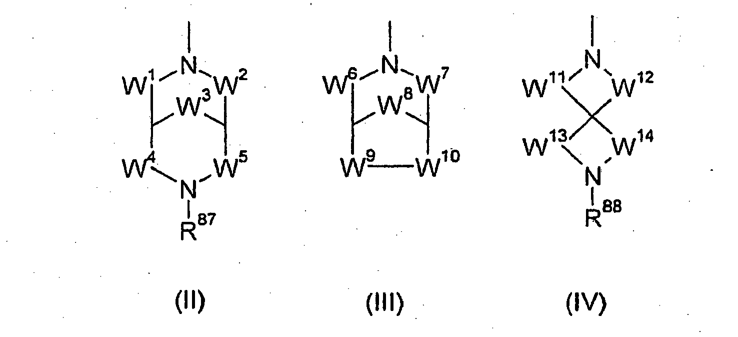 worin bedeuten
W1, W2, W3, W4, W5, W6, W7, W8, W9, W10, W11, W12, W13, W14
unabhängig voneinander eine Bindung, C=C, 1 bis 4-gliedrige Alkylen- oder Alkylidenkette, in der 0-1 Kohlenstoffatome außerhalb einer in der Alkylidenkette vorhandenen Doppelbindung durch ein Element aus der Gruppe N(R90), O und S ersetzt sein können;
wobei die Kohlenstoffatome in den Gruppen der Formeln (II), (III) und (IV) substituiert sein können mit H, F, OH, Oxo, (C₁-C₆)-Alkyl, O-(C₁-C₆)-Alkyl, (CR52R53)ₒR54, bevorzugt H, (CR52R53)ₒR54;
R87, R88, R90 H, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
R56, R57, R58, R59, R62, R63 unabhängig voneinander H, -(C₁-C₈)-Alkyl;
oder
R56 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R60, R61 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78). SO₂Me;
R64, R65, R66, R68, R69, R71,
R72, R73, R74, R75, R76, R77, R78 unabhängig voneinander H, (C₁-C₈)-Alkyl;
oder
R76 und R77 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
deren N-Oxide sowie deren physiologisch verträgliche Salze.

3. Verbindungen nach Anspruch 2, worin Q die folgenden Bedeutungen aufweist: 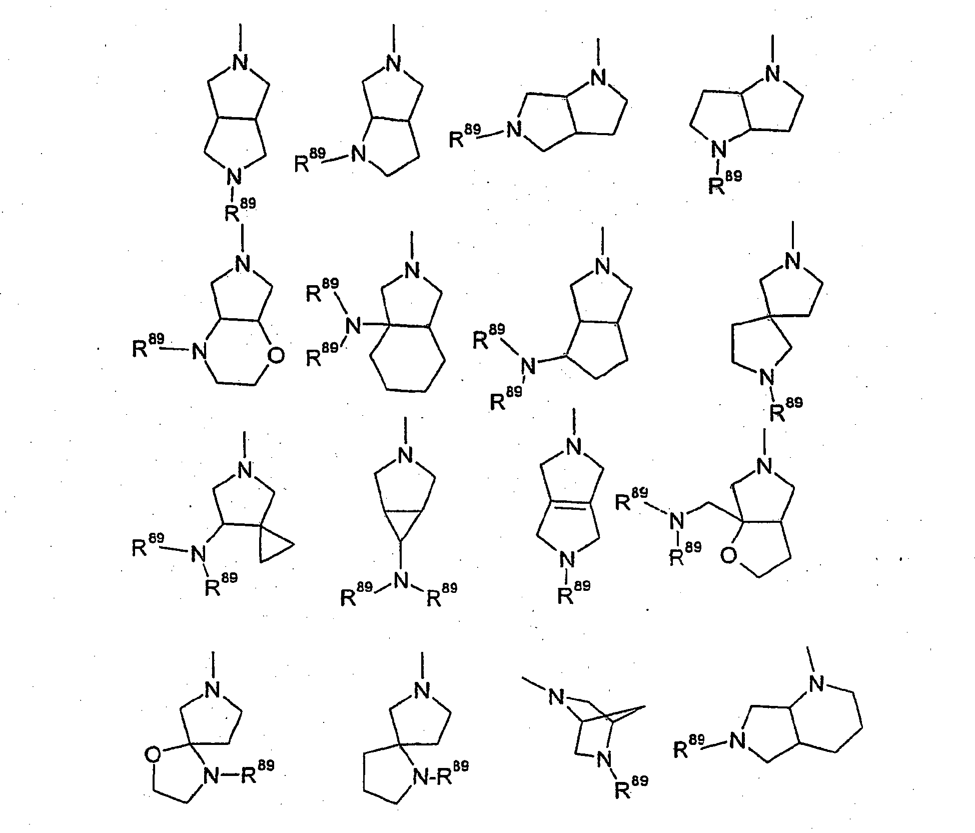 worin bedeuten:
R89, in der Gruppierung N-R89: H, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55; bevorzugt H, (C₁-C₆)-Alkyl;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
R56, R57, R58, R59, R62, R63 unabhängig voneinander H, (C₁-C₈)-Alkyl;
oder
R56 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R60, R61 unabhängig voneinander H, (C₁-C₆)-Alkyl, (C1-C4)-Alkoxy-(C1-C4)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, -(C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 unabhängig voneinander H, (C₁-C₈)-Alkyl;
oder
R76 und R77 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, wekher außer dem Stickstoffatom noch 0-1 weitere Heteroatome aus der Gruppe NH, N-(C₁-C₆)-Alkyl, Sauerstoff und Schwefel beinhalten kann;
R89 ind der Gruppierung N(R89)₂:
unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R⁶⁷, CO(CR68R69)ᵣ-R70; oder die beiden Reste R89 bilden zusammen mit dem Stickstoffatom, an das sie gebunden sind, einen 4 bis 10-gliedrigen mono-, bi- oder spirocyclischen Ring, welcher außer dem dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me; bevorzugt H, (C₁-C₆)-Alkyl, oder die beiden Reste R89 bilden zusammen mit dem Stickstoffatom, an das sie gebunden sind, einen 5 bis 6-gliedrigen monocyclischen Ring, welcher außer dem Stickstoffatom 0 bis 1 zusätzliches Heteroatom ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me.

4. Verbindungen der Formel I gemäß einem der Ansprüche 1 bis 3, **dadurch**
**gekennzeichnet, dass**
A, B, D, G unabhängig voneinander N oder C(R3) bedeuten und die Gesamtzahl der Stickstoffatome in diesem Ring 0-2, bevorzugt 0 oder 1, ganz besonders bevorzugt 0 beträgt.

5. Verbindungen der Formel I, gemäß einem der Ansprüche 1 bis 4, worin bedeuten
K Bindung, O, CO, OCH₂, CH₂O, N(R36)CO, CON(R37), (C(R38)(R39))₂, (R31)C=C(R32), C≡C, SCH₂, SO₂CH₂, besonders bevorzugt OCH₂, CH₂O, N(R36)CO, CON(R37), (C(R38)(R39))₂, (R31)C=C(R32), C≡C, SCH₂, SO₂CH₂, ganz besonders bevorzugt OCH₂, CH₂O, CON(R37), (C(R38)(R39))₂, C≡C, SCH₂; wobei
R31, R32, R36, R37, R38, R39 unabhängig voneinander H, (C₁-C₈)-Alkyl bedeuten.

6. Verbindungen nach einem der Ansprüche 1 bis 5 worin bedeuten
R2 (C₁-C₈)-Alkyl, (C₁-C₄)-Alkoxy-(C₁-C₄)-alkyl" ein 3 bis 10-gliedriger mono-, bi-, tri- oder spirocyclischer Ring, welcher 0 bis 3 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, (C₀-C₂)-Alkylen-aryl, Oxo, CO(R41), CON(R42)(R43), Hydroxy, N(R45)CO(C₁-C₆)-Alkyl, N(R46)(R47) oder SO₂CH₃; bevorzugt (C₁-C₈)-Alkyl, (C₁-C₄)-Alkoxy-(C₁-C₄)-alkyl, ein 3 bis 10-gliedriger mono- oder bicyclischer Ring, welcher 0 bis 2 Heteroatome beinhalten kann, ausgewählt aus der Gruppe Sauerstoff, Stickstoff und Schwefel, wobei das Ringsystem zusätzlich substituiert sein kann mit einem oder mehreren der folgenden Substituenten: F, Cl, Br, CF₃, CN, (C₁-C₆)-Alkyl, O-(C₁-C₈)-Alkyl, Oxo, CO(R41), CON(R42)(R43), N(R45)CO(C₁-C₆)-Alkyl oder SO₂CH₃;
R41, R42, R43, R45, R46, R47 unabhängig voneinander H, (C₁-C₈)-Alkyl;
oder R42 und R43, R46 und R47 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

7. Verbindungen der Formel I nach einem der Ansprüche 1 bis 6, worin bedeuten
A, B, D, G C(R3);
R3 H, F, Cl, Br, CF₃, CN, O-(C₁-C₆)-Alkyl, O-(C₁-C₄)-Alkoxy-(C₁-C₄)-alkyl, S-(C₁-C₆)-Alkyl, (C₁-C₆)-Alkyl, (C₀-C₈)-Alkylen-aryl, O-(C₀ - C₈)-Alkylen-aryl, N(R4)(R5), SO₂-CH₃, CON(R6)(R7), N(R8)CO(R9), CO(R12), (CR13R14)ₓ-O(R15); bevorzugt H, F, Cl, Br, CF₃, CN, O-(C₁-C₆)-Alkyl, (C₁-C₆)-Alkyl, SO₂-CH₃, CON(R6)(R7), N(R8)CO(R9), CO(R12), (CR13R14)ₓ-O(R15), besonders bevorzugt H, F, Cl, CF₃, CN, (C₁-C₆)-Alkyl, (C(R13)(R14))ₓ-O(R15); ganz besonders bevorzugt H, F, Cl, (C₁-C₆)-Alkyl; ganz ganz besonders bevorzugt H, CH₃, F;
R4, R5, R6, R7, R8 unabhängig voneinander H, (C₁-C₈)-Alkyl;
oder
R4 und R5, R6 und R7 unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R9, R12 unabhängig voneinander H, (C₁-C₈)-Alkyl;
R13, R14 H;
R15 H, (C₁-C₆)-Alkyl;
x 0, 1, 2, bevorzugt 0, 1, besonders bevorzugt 1.

8. Verbindungen nach Anspruch 1 worin bedeuten
Q bi-, tri- oder spirocyclisches gesättigtes oder teilweise ungesättigtes Ringgerüst mit einem Stickstoffatom und 0-3 weiteren Heteroatomen ausgewählt aus der Gruppe N, O und S, wobei die Ringe des Gerüsts spiroverknüpft, kondensiert oder verbrückt sein können, und wobei das Ringsystem substituiert sein kann mit einem oder mehreren der folgenden Substituenten: F, OH, CF₃, CN, OCF₃, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), OH;
R56, R57, R58, R59, R62, R63 unabhängig voneinander H, (C₁-C₈)-Alkyl;
oder
R56 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R60, R61 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 unabhängig voneinander H, (C₁-C₈)-Alkyl;
oder
R76 und R77 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

9. Verbindungen nach Anspruch 1 worin bedeuten
Q bi-, tri- oder spirocyclisches gesättigtes Ringgerüst mit einem Stickstoffatom und 0-3 weiteren Heteroatomen ausgewählt aus der Gruppe N, O und S, wobei die Ringe des Gerüsts spiro-verknüpft, kondensiert oder verbrückt sein können, und wobei das Ringsystem substituiert sein kann mit einem oder mehreren der folgenden Substituenten: F, OH, CF₃, CN, OCF₃, Oxo, O-(C₁-C₈)-Alkyl, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60)(R61), CO₂(R62), SO₂Me, CN, ein 3-10 gliedriges Ringsystem mit 0 bis 3 Heteroatomen ausgewählt aus der Gruppe N, O und S, das substituiert sein kann mit F, Cl, Br, CF₃, (C₁-C₈)-Alkyl, O-(C₁-C₈)-Alkyl, CO(R63), Oxo, OH; unabhängig voneinander H, (C₁-C₈)-Alkyl;
oder
R66 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R60, R61 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 unabhängig voneinander H, (C₁-C₈)-Alkyl;
oder
R76 und R77 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann.

10. Verbindungen nach einem der Ansprüche 2 bis 9, **dadurch gekennzeichnet, dass** W3 in Formel II und W8 in Formel III jeweils eine Bindung bedeuten.

11. Verbindungen nach Anspruch 9, worin Q die folgenden Bedeutungen aufweist 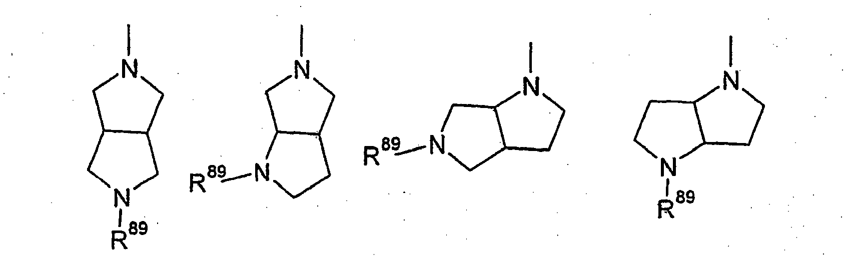 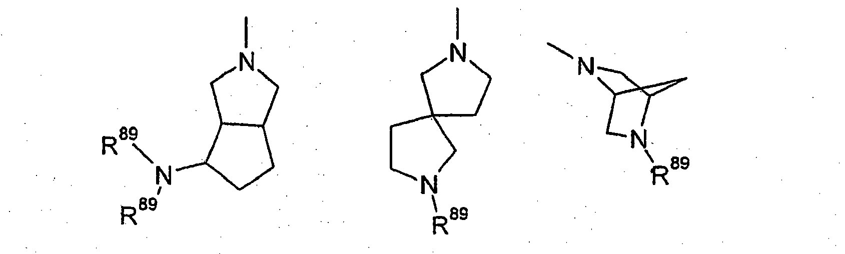 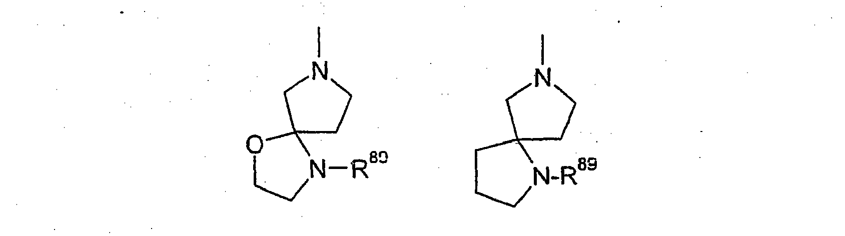 worin bedeuten:
R89, in der Gruppierung N-R89: H, (C₁-C₄)-Alkoxy-(C₁-C₄)-alkyl, (C₁-C₆)-Alkyl, (C₂-C₆)-Alkenyl, (C₂-C₆)-Alkinyl, CO(R51), (CR52R53)ₒ-R54, CO(CR52R53)ₚ-R55; bevorzugt H, (C₁-C₆)-Alkyl;
R51 H, (C₁-C₈)-Alkyl;
R52, R53 unabhängig voneinander H, (C₁-C₈)-Alkyl, OH, (C₃-C₈)-Cycloalkyl, (C₁-C₄)-Alkoxy-(C₁-C₄)-alkyl;
o, p unabhängig voneinander 0, 1, 2, 3, 4, 5, 6;
R54, R55 unabhängig voneinander OH, O-(C₁-C₈)-Alkyl, CON(R56)(R57), N(R58)CO(R59), N(R60XR61), CO₂(R62), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R63), Oxo, OH;
R56, R57, R58, R59, R62, R63 unabhängig voneinander H, (C₁-C₈)-Alkyl;
oder
R56 und R57 bilden optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R60, R61 unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), - (CR65R66)q-R67, CO(CR68R69)ᵣ-R70; oder R60 und R61 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO2(R78), SO₂Me;
R64, R65, R66, R68, R69, R71,
R72, R73, R74, R75, R76, R77, R78 unabhängig voneinander H, (C₁-C₈)-Alkyl;
oder
R76 und R77 optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
q, r unabhängig voneinander 0, 1, 2, 3, 4, 5, 6;
R67, R70 unabhängig voneinander OH, O-(C₁-C₈)-Alkyl, CON(R79)(R80), N(R81)CO(R82), N(R83)(R84), CO₂(R85), SO₂Me, CN, ein 3-10 gliedriges Ringsystem mit 0 bis 3 Heteroatomen ausgewählt aus der Gruppe N, O und S, das substituiert sein kann mit einem oder mehreren der folgenden Substituenten: F, Cl, Br, CF₃, (C₁-C₈)-Alkyl, O-(C₁-C₈)-Alkyl, CO(R86), Oxo, OH;
R79, R80, R81, R82, R83, R84, R85, R86 unabhängig voneinander H, (C₁-C₈)-Alkyl;
oder
R79 und R80, R83 und R84 bilden unabhängig voneinander optional zusammen mit dem Stickstoffatom, an das sie gebunden sind, einen 5-6 gliedrigen Ring, welcher außer dem Stickstoffatom noch 0-1 weitere Heteroatome aus der Gruppe NH, N-(C₁-C₆)-Alkyl, Sauerstoff und Schwefel beinhalten kann;
R89 in der Gruppierung N(R89)₂: unabhängig voneinander H, (C₁-C₆)-Alkyl, (C₁-C₄)-Alkoxy-(C₁-C₄)-alkyl, (C₂-C₆)-Alkenyl, (C₂-C₆)-Alkinyl, CO(R64), (CR65R66)_{q}-R67, CO(CR68R69)ᵣ-R70; oder die beiden Reste R89 bilden zusammen mit dem Stickstoffatom, an das sie gebunden sind, einen 4 bis 10-gliedrigen mono-, bi- oder spirocyclischen Ring, welcher außer dem Stickstoffatom 0 bis 3 zusätzliche Heteroatome ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me; bevorzugt H, (C₁-C₆)-Alkyl, oder die beiden Reste R89 bilden zusammen mit dem Stickstoffatom, an das sie gebunden sind, einen 5 bis 6-gliedrigen monocyclischen Ring, welcher außer dem Stickstoffatom 0 bis 1 zusätzliches Heteroatom ausgewählt aus der Gruppe N, O und S enthält und zusätzlich substituiert sein kann mit einem oder mehreren der folgenden Substituenten: F, Cl, Br, CF₃, O-(C₁-C₈)-Alkyl, (C₁-C₆)-Alkyl, CO(R71), Oxo, OH, (C₁-C₄)-Alkoxy-(C₁-C₄)-alkyl, Hydroxy-(C₁-C₄)-alkyl, CON(R72)(R73), N(R74)CO(R75), N(R76)(R77), CO₂(R78), SO₂Me.

12. Verbindungen nach einem der Ansprüche 1 bis 11, **dadurch gekennzeichnet, dass** X Bindung oder N(R16) bedeutet, worin R16 H oder (C₁-C₈)-Alkyl bedeutet.

13. Arzneimittel enthaltend eine oder mehrere der Verbindungen der Formel I gemäß einem oder mehreren der Ansprüche 1 bis 12.

14. Arzneimittel enthaltend eine oder mehrere der Verbindungen der Formel I gemäß einem oder mehreren der Ansprüche 1 bis 12 und einen oder mehrere Wirkstoffe, die günstige Wirkungen auf Stoffwechselstörungen oder damit assozierte Erkrankungen haben.

15. Arzneimittel enthaltend eine oder mehrere der Verbindungen der Formel I gemäß einem oder mehreren der Ansprüche 1 bis 12 und ein oder mehrere Antidiabetika.

16. Arzneimittel enthaltend eine oder mehrere der Verbindungen der Formel I gemäß einem oder mehreren der Ansprüche 1 bis 12 und einen oder mehrere Lipidmodulatoren.

17. Verwendung der Verbindungen der Formel I gemäß einem oder mehreren der Ansprüche 1 bis 12 zur Herstellung eines Arzneimittels zur Behandlung und/oder Prävention von Störungen des Fettsäurestoffwechsels und Glucoseverwertungsstörungen.

18. Verwendung der Verbindungen der Formel I gemäß einem oder mehreren der Ansprüche 1 bis 12 zur Herstellung eines Arzneimittels zur Behandlung und/oder Prävention von Störungen, bei denen Insulin Resistenz eine Rolle spielt.

19. Verwendung der Verbindungen der Formel I gemäß einem oder mehreren der Ansprüche 1 bis 12 zur Herstellung eines Arzneimittels zur Behandlung und/oder Prävention von Diabetes mellitus und der damit verbundenen Folgeerkrankungen.

20. Verwendung der Verbindungen der Formel I gemäß einem oder mehreren der Ansprüche 1 bis 12 zur Herstellung eines Arzneimittels zur Behandlung und/oder
Prävention von Dyslipidämien und deren Folgen.

21. Verwendung der Verbindungen der Formel I gemäß einem oder mehreren der Ansprüche 1 bis 12 zur Herstellung eines Arzneimittels zur Behandlung und/oder Prävention von Zuständen, die mit dem Metabolischen Syndrom assoziert sind.

22. Verwendung der Verbindungen der Formel 1 gemäß einem oder mehreren der Ansprüche 1 bis 12 zur Herstellung eines Arzneimittels zur Behandlung und/oder Prävention von Obesitas und damit verbundenen Folgeerkrankungen.

23. Verwendung der Verbindungen gemäß einem oder mehreren der Ansprüche 1 bis 12 in Kombination mit mindestens einem weiteren Wirkstoff zur Herstellung eines Arzneimittels zur Behandlung und/oder Prävention von Störungen des Fettsäurestoffwechsels und Glucoseverwertungsstörungen.

24. Verwendung der Verbindungen gemäß einem oder mehreren der Ansprüche 1 bis 12 in Kombination mit mindestens einem weiteren Wirkstoff zur Herstellung eines Arzneimittels zur Behandlung und/oder Prävention von Störungen, bei denen Insulin Resistenz eine Rolle spielt.

25. Verfahren zur Herstellung eines Arzneimittels enthaltend eine oder mehrere der Verbindungen gemäß einem oder mehreren der Ansprüche 1 bis 12, **dadurch gekennzeichnet, dass** der Wirkstoff mit einem pharmazeutisch geeigneten Träger vermischt wird und diese Mischung in eine für die Verabreichung geeignete Form gebracht wird.

## Claims

1. A compound of the formula I 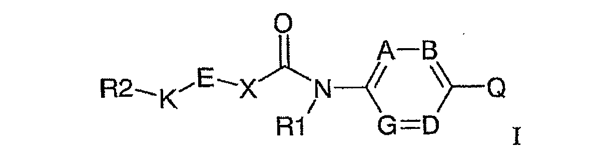 in which the meanings are
A, B, D, G independently of one another N, C(R3) or groups A and B or D and G are each C(R3) and form together an ortho-phenylene unit to result overall in a 1,4-disubstituted naphthalene system; preferably independently of one another N or C(R3), where the total number of nitrogen atoms in the ring is 0 - 2, preferably 0 or 1, and particularly preferably C(R3);
R3 H, F, Cl, Br, CF₃, CN, O-(C₁-C₆)-alkyl, O-(C₁-C₄)-alkoxy-(C₁-C₄)-alkyl, S-(C₁-C₆)-alkyl, (C₁-C₆)-alkyl, (C₀-C₈)-alkylene-aryl, O-(C₀ - C₈)-alkylene-aryl, N(R4)(R5), SO₂-CH₃, CON(R6)(R7), N(R8)CO(R9), CO(R12), (CR13R14)ₓ-O(R15); preferably H, F, Cl, Br, CF₃, CN, O-(C₁-C₆)-alkyl, (C₁-C₆)-alkyl, SO₂-CH₃, CON(R6)(R7), N(R8)CO(R9), CO(R12), (CR13R14)ₓ-O(R15), particularly preferably H, F, Cl, CF₃, CN, (C₁-C₆)-alkyl, (C(R13)(R14))ₓ-O(R15); very particularly preferably H, F, CL, (C₁-C₆)-alkyl;
R4, R5, R6, R7, R8 independently of one another H, (C₁-C₈)-alkyl;
or
R4 and R5, R6 and R7 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R9, R12 independently of one another H, (C₁-C₈)-alkyl;
R13, R14 H;
R15 H, (C₁-C₆)-alkyl;
x 0, 1, 2, preferably 0, 1, particularly preferably 1;
R1 H, (C₁-C₈)-alkyl;
X N(R16), a bond, (R17)C=C(R18), C=C, CH₂-CH₂, YCH₂, CH₂Y, preferably N(R16), a bond;
Y O, S, N(R21);
R16, R17, R18 independently of one another H, (C₁-C₈)-alkyl; preferably H;
R21 H, (C₁-C₈)-alkyl;
E 3-8 membered bivalent carbo- or heterocyclic ring structure having 0-4 heteroatoms from the group of N, O and S, which may optionally have substituents from the group of H, F, Cl, Br, OH, CF₃, NO₂, CN, OCF₃, O-(C₁-C₆)-alkyl, O-(C₁-C₄)-alkoxy-(C₁-C₄)-alkyl, S-(C₁-C₆)-alkyl, (C₁-C₆)-alkyl, (C₂-C₆)-alkenyl, O-(C₃-C₈)-cycloalkyl, (C₃-C₈)-cycloalkenyl, (C₂-C₆)-alkynyl, (C₀-C₈)-alkylene-aryl, O-(C₀-C₈)-alkylene-aryl, S-aryl, N(R22)(R23), SO₂-CH₃, N(R26)CO(R27), N(R28)SO₂(R29), CO(R30) and be mono- or bicyclic; preferably 5-7 membered bivalent carbo- or heterocyclic ring structure having 0-3 heteroatoms from the group of N, O and S, which may optionally have substituents from the group of H, F, Cl, Br, OH, CF₃, NO₂, CN, OCF₃, O-(C₁-C₆)-alkyl, S-(C₁-C₆)-alkyl, (C₁-C₆)-alkyl, (C₂-C₆)-alkenyl, O-(C₀-C₈)-alkylene-aryl, S-aryl, N(R22)(R23), SO₂-CH₃, N(R26)CO(R27), CO(R30) and be mono- or bicyclic; particularly preferably 5-7 membered bivalent carbo- or heterocyclic ring structure having 0-2 heteroatoms from the group of N, O and S, which may optionally have substituents from the group of H, F, Cl, Br, OH, CF₃, NO₂, OCF₃, O-(C₁-C₆)-alkyl, (C₁-C₆)-alkyl, (C₂-C₆)-alkenyl, N(R22)(R23), SO₂-CH₃, CO(R30), preferably H, F, Cl, Br, OH, CF₃, (C₁-C₆)-alkyl, O-(C₁-C₆)-alkyl, e.g. E is selected from the group consisting of 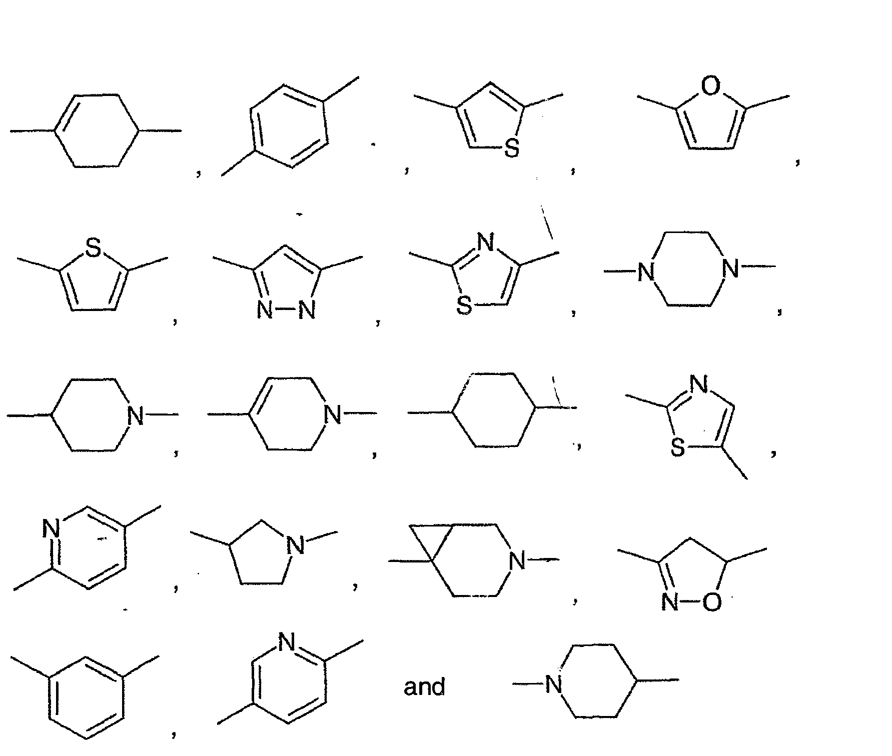 which may optionally have substituents from the group of H, F, Cl, Br, OH, CF₃, NO₂, OCF₃, O-(C₁-C₆)-alkyl, (C₁-C₆). alkyl, (C₂-C₆)-alkenyl, N(R22)(R23), SO₂-CH₃, CO(R30), preferably H, F, Cl, Br, OH, CF₃, (C₁-C₆)-alkyl, O-(C₁-C₆)-alkyl; preferably 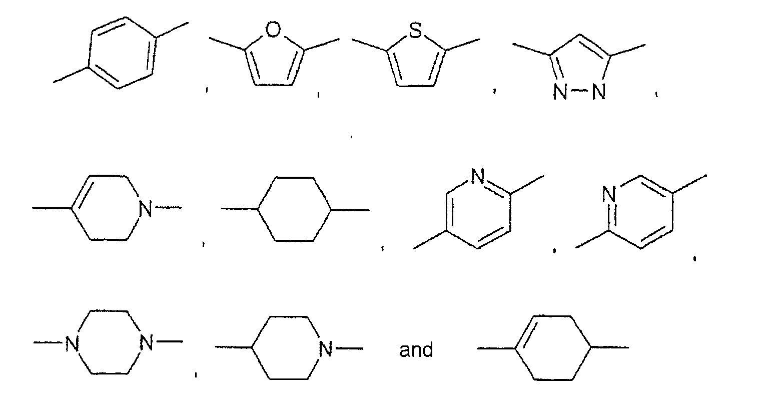 which may optionally have the aforementioned substituents;
R22, R23, R24, R25, R26, R28 independently of one another H, (C₁-C₈)-alkyl;
or
R22 and R23, R24 and R25 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R27, R29, R30 independently of one another H, (C₁-C₈)-alkyl;
K a bond, O, OCH₂, CH₂O, S, SO, SO₂, N(R35), N(R36)CO, CON(R37), (C(R38)(R39))ᵥ, CO, (R31)C=C(R32), C≡C, SCH₂, SO₂CH₂, particularly preferably OCH₂, CH₂O, N(R36)CO, CON(R37), (C(R38)(R39))₂, (R31)C=C(R32), C= C, SCH₂, SO₂CH₂, very particularly preferably OCH₂, CH₂O, CON(R37), C≡C, SCH₂;
v 2;
R31, R32, R35, R36, R37, R38, R39 independently of one another H, (C₁-C₈)-alkyl;
R2 (C₁-C₈)-alkyl, (C₁-C₄)-alkoxy-(C₁-C₄)-alkyl, a 3 to 10-membered mono-, bi-, tri- or spirocyclic ring which may include 0 to 3 heteroatoms selected from the group of oxygen, nitrogen and sulfur, where the ring system may additionally be substituted by one or more of the following substituents: F, Cl, Br, CF₃, CN, (C₁-C₆)-alkyl, O-(C₁-C₈)-alkyl, (C₀-C₂)-alkylene-aryl, oxo, CO(R41), CON(R42)(R43), hydroxy, N(R45)CO(C₁-C₆)-alkyl, N(R46)(R47) or SO₂CH₃; preferably (C₁-C₈)-alkyl, (C₁-C₄)-alkoxy-(C₁-C₄)-alkyl, a 3 to 10-membered mono- or bicyclic ring which may include 0 to 2 heteroatoms selected from the group of oxygen, nitrogen and sulfur, where the ring system may additionally be substituted by one or more of the following substituents: F, Cl, Br, CF₃, CN, (C₁-C₆)-alkyl, O-(C₁-C₈)-alkyl, oxo, CO(R41), CON(R42)(R43), N(R45)CO(C₁-C₆)-alkyl or SO₂CH₃;
R41, R42, R43, R45, R46, R47 independently of one another H, (C₁-C₈)-alkyl;
or
R42 and R43, R46 and R47 form independently of one another optional together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
Q bi-, tri- or spirocyclic saturated or partially unsaturated ring structure having a nitrogen atom and 0-3 further heteroatoms selected from the group of N, O and S, where the rings of the structure may be spiro-linked, fused or bridged, and where the ring system may be substituted by one or more of the following substituents: F, OH, CF₃, CN, OCF₃, oxo, O-(C₁-C₈)-alkyl, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)ₒ-R54, CO(CR52R53)ₚ-R55;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO₂(R62), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R63), oxo, OH;
R56, R57, R58, R59, R62, R63 independently of one another H, (C₁-C₈)-alkyl;
or
R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 independently of one another H, (C₁-C₈)-alkyl;
or
R76 and R77 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur,
the N-oxides thereof and the physiologically tolerated salts thereof.

2. A compound as claimed in claim 1, in which Q has the following meanings: 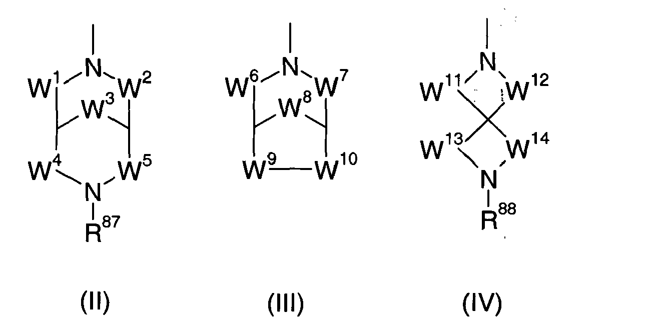 in which the meanings are
W1, W2, W3, W4, W5, W6, W7,
W8, W9, W10, W11, W12, W13, W14 independently of one another a bond, C=C, 1 to 4-membered alkylene or alkylidene chain in which 0-1 carbon atoms outside a double bond present in the alkylidene chain may be replaced by an element from the group of N(R90), O and S; where the carbon atoms in the groups of the formulae (II), (III) and (IV) may be substituted by H, F, OH, oxo, (C₁-C₆)-alkyl, O-(C₁-C₆)-alkyl, (CR52R53)ₒR54, preferably H, (CR52R53)ₒR54;
R87, R88, R90 H, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)ₒ-R54, CO(CR52R53)ₚ-R55;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO₂(R62),SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R63), oxo, OH;
R56, R57, R58, R59, R62, R63 independently of one another H, (C₁-C₈)-alkyl;
or
R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 independently of one another H, (C₁-C₈)-alkyl;
or
R76 and R77 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
the N-oxides thereof and the physiologically tolerated salts thereof.

3. A compound as claimed in claim 2, in which Q has the following meanings: 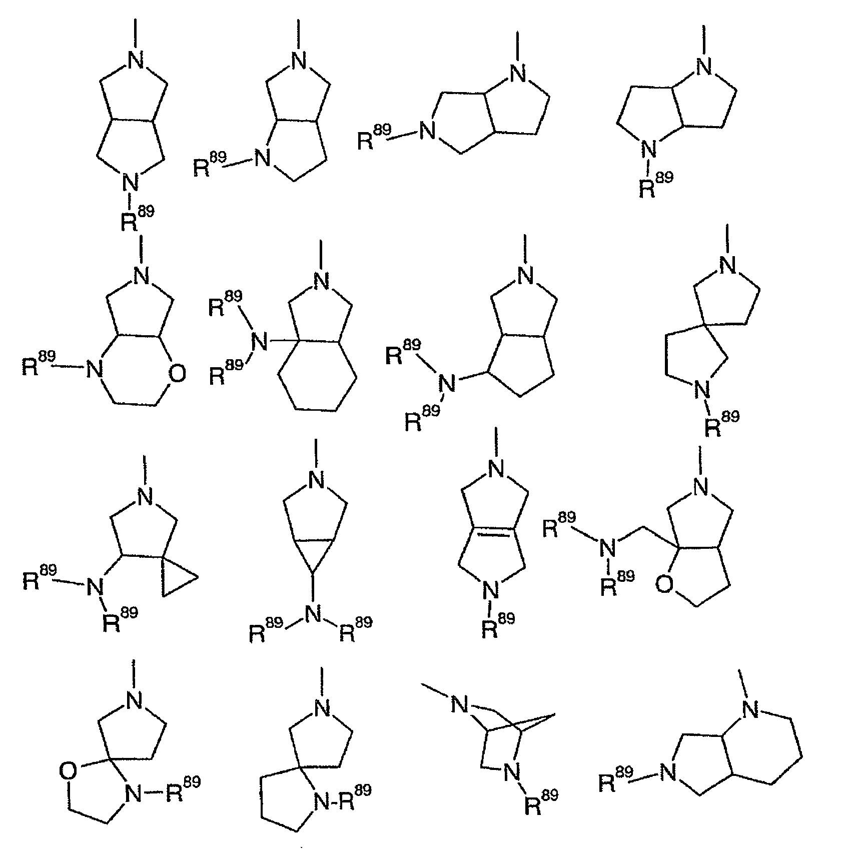 in which the meanings are:
R89 in the group N-R89: H, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)ₒ-R54, CO(CR52R53)ₚ-R55; preferably H, (C₁-C₆)-alkyl;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO₂(R62), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R63), oxo, OH;
R56, R57, R58, R59, R62, R63 independently of one another H, (C₁-C₈)-alkyl;
or
R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me;
R64, R65, R66, R68, R69,
R71, R72, R73, R74, R75, R76, R77, R78 independently of one another H, (C₁-C₈)-alkyl;
or
R76 and R77 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R89 in the group N(R89)₂: independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or the two R89 radicals form together with the nitrogen atom to which they are bonded a 4 to 10-membered mono-, bi- or spirocyclic ring, which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me; preferably H, (C₁-C₆)-alkyl, or the two R89 radicals form together with the nitrogen atom to which they are bonded a 5 to 6-membered monocyclic ring which, apart from the nitrogen atom, comprises 0 to 1 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me.

4. A compound of the formula I as claimed in any of claims 1 to 3,
wherein
A, B, D, G are independently of one another N or C(R3), and the total number of nitrogen atoms in this ring is 0-2, preferably 0 or 1, very particularly preferably 0.

5. A compound of the formula I as claimed in any of claims 1 to 4, in which the meanings are
K bond, O, CO, OCH₂, CH₂O, N(R36)CO, CON(R37), (C(R38)(R39))₂, (R31)C=C(R32), C≡C, SCH₂, SO₂CH₂, particularly preferably OCH₂, CH₂O, N(R36)CO, CON(R37), (C(R38)(R39))₂, (R31)C=C(R32), C≡C, SCH₂, SO₂CH₂, very particularly preferably OCH₂, CH₂O, CON(R37), (C(R38)(R39))₂, C= C, SCH₂; where
R31, R32, R36, R37, R38, R39 are independently of one another H, (C₁-C₈)-alkyl.

6. A compound as claimed in any of claims 1 to 5 in which the meanings are
R2 (C₁-C₈)-alkyl, (C₁-C₄)-alkoxy-(C₁-C₄)-alkyl, a 3 to 10-membered mono-, bi-, tri- or spirocyclic ring which may include 0 to 3 heteroatoms selected from the group of oxygen, nitrogen and sulfur, where the ring system may additionally be substituted by one or more of the following substituents: F, Cl, Br, CF₃, CN, (C₁-C₆)-alkyl, O-(C₁-C₈)-alkyl, (C₀-C₂)-alkylene-aryl, oxo, CO(R41), CON(R42)(R43), hydroxy, N(R45)CO(C₁-C₆)-alkyl, N(R46)(R47) or SO₂CH₃; preferably (C₁-C₈)-alkyl, (C₁-C₄)-alkoxy-(C₁-C₄)-alkyl, a 3 to 10-membered mono- or bicyclic ring which may include 0 to 2 heteroatoms selected from the group of oxygen, nitrogen and sulfur, where the ring system may additionally be substituted by one or more of the following substituents: F, Cl, Br, CF₃, CN, (C₁-C₆)-alkyl, O-(C₁-C₈)-alkyl, oxo, CO(R41), CON(R42)(R43), N(R45)CO(C₁-C₆)-alkyl or SO₂CH₃;
R41, R42, R43, R45, R46, R47 independently of one another H, (C₁-C₈)-alkyl;
or
R42 and R43, R46 and R47 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur.

7. A compound of the formula I as claimed in any of claims 1 to 6, in which the meanings are
A, B, D, G C(R3);
R3 H, F, Cl, Br, CF₃, CN, O-(C₁-C₆)-alkyl, O-(C₁-C₄)-alkoxy-(C₁-C₄)-alkyl, S-(C₁-C₆)-alkyl, (C₁-C₆)-alkyl, (C₀-C₈)-alkylene-aryl, O-(C₀-C₈)-alkylene-aryl, N(R4)(R5), SO₂-CH₃, CON(R6)(R7), N(R8)CO(R9), CO(R12), (CR13R14)ₓ-O(R15); preferably H, F, Cl, Br, CF₃, CN, O-(C₁-C₆)-alkyl, (C₁-C₆)-alkyl, SO₂-CH₃, CON(R6)(R7), N(R8)CO(R9), CO(R12), (CR13R14)ₓ-O(R15), particularly preferably H, F, Cl, CF₃, CN, (C₁-C₆)-alkyl, (C(R13)(R14))ₓ-O(R15); very particularly preferably H, F, Cl, (C₁-C₆)-alkyl; very very particularly preferably H, CH₃, F;
R4, R5, R6, R7, R8 independently of one another H, (C₁-C₈)-alkyl;
or
R4 and R5, R6 and R7 independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R9, R12 independently of one another H, (C₁-C₈)-alkyl;
R13, R14 H;
R15 H, (C₁-C₆)-alkyl;
x 0, 1, 2, preferably 0, 1, particularly preferably 1.

8. A compound as claimed in claim 1 in which the meanings are
Q bi-, tri- or spirocyclic saturated or partially unsaturated ring structure having one nitrogen atom and 0-3 further heteroatoms selected from the group of N, O and S, where the rings of the structure may be spiro-linked, fused or bridged, and where the ring system may be substituted by one or more of the following substituents: F, OH, CF₃, CN, OCF₃, O-(C₁-C₈)-alkyl, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)ₒ-R54, CO(CR52R53)ₚ-R55;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO₂(R62), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R63), OH;
R56, R57, R58, R59, R62, R63 independently of one another H, (C₁-C₈)-alkyl;
or
R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 independently of one another H, (C₁-C₈)-alkyl;
or
R76 and R77 form optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur.

9. A compound as claimed in claim 1 in which the meanings are
Q bi-, tri- or spirocyclic saturated ring structure having one nitrogen atom and 0-3 further heteroatoms selected from the group of N, O and S, where the rings of the structure may be spiro-linked, fused or bridged, and where the ring system may be substituted by one or more of the following substituents: F, OH, CF₃, CN, OCF₃, oxo, O-(C₁-C₈)-alkyl, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)ₒ-R54, CO(CR52R53)ₚ-R55;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO₂(R62),SO₂Me, CN, a 3-10 membered ring system having 0 to 3 heteroatoms selected from the group of N, O and S, which may be substituted by F, Cl, Br, CF₃, (C₁-C₈)-alkyl, O-(C₁-C₈)-alkyl, CO(R63), oxo, OH;
R56, R57, R58, R59, R62, R63 independently of one another H, (C₁-C₈)-alkyl;
or
R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me;
R64, R65, R66, R68, R69, R71,
R72, R73, R74, R75, R76, R77, R78 independently of one another H, (C₁-C₈)-alkyl;
or
R76 and R77 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl,oxygen and sulfur.

10. A compound as claimed in any of claims 2 to 9, wherein W3 in formula II and W8 in formula III are each a bond.

11. A compound as claimed in claim 9, in which Q has the following meanings 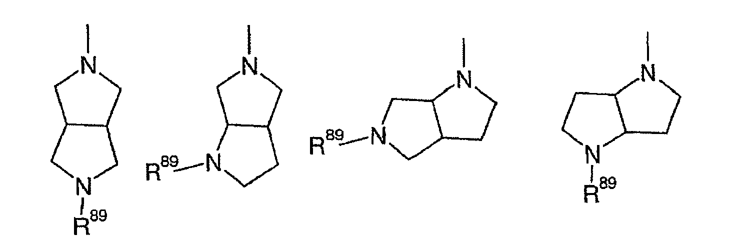 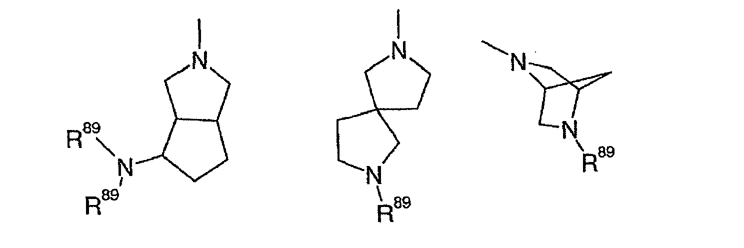 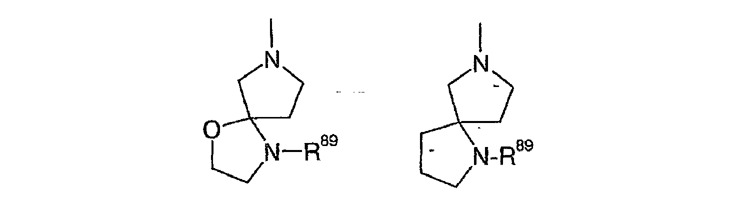 in which the meanings are:
R89 in the group N-R89: H, (C₁-C₄)-alkoxy-(C₁-C₄)-alkyl, (C₁-C₆)-alkyl, (C₂-C₆)-alkenyl, (C₂-C₆)-alkynyl, CO(R51), (CR52R53)o-R54, CO(CR52R53)p-R55; preferably H, (C₁-C₆)-alkyl;
R51 H, (C₁-C₈)-alkyl;
R52, R53 independently of one another H, (C₁-C₈)-alkyl, OH, (C₃-C₈)-cycloalkyl, (C₁-C₄)-alkoxy-(C₁-C₄)alkyl;
o, p independently of one another 0, 1, 2, 3, 4, 5, 6;
R54, R55 independently of one another OH, O-(C₁-C₈)-alkyl, CON(R56)(R57), N(R58)CO(R59), N(R60)(R61), CO2(R62), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R63), oxo, OH;
R56, R57, R58, R59, R62, R63 independently of one another H, (C₁-C₈)-alkyl;
or R56 and R57 form optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R60, R61 independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or R60 and R61 form together with the nitrogen atom to which they are bonded a 4 to 10-membered mono-, bi- or spirocyclic ring which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO2(R78), SO₂Me;
R64, R65, R66, R68, R69, R71,
R72, R73, R74, R75, R76, R77, R78 independently of one another H, (C₁-C₈)-alkyl; or
R76 and R77 optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
q, r independently of one another 0, 1, 2, 3, 4, 5, 6;
R67, R70 independently of one another OH, O-(C₁-C₈)-alkyl, CON(R79)(R80), N(R81)CO(R82), N(R83)(R84), CO₂(R85), SO₂Me, CN, a 3-10 membered ring system having 0 to 3 heteroatoms selected from the group of N, O and S, which may be substituted by one or more of the following substituents: F, Cl, Br, CF₃, (C₁-C₈)-alkyl, O-(C₁-C₈)-alkyl, CO(R86), oxo, OH;
R79, R80, R81, R82, R83, R84, R85, R86 independently of one another H, (C₁-C₈)-alkyl;
or
R79 and R80, R83 and R84 form independently of one another optionally together with the nitrogen atom to which they are bonded a 5-6 membered ring which, apart from the nitrogen atom, may also include 0-1 further heteroatoms from the group of NH, N-(C₁-C₆)-alkyl, oxygen and sulfur;
R89 in the group N(R89)₂: independently of one another H, (C₁-C₆)-alkyl, (C₁-C₄)-alkoxy-(C₁-C₄)-alkyl, (C₂-C₆)-alkenyl, (C₂-C₆)-alkynyl, CO(R64), (CR65R66)q-R67, CO(CR68R69)ᵣ-R70; or the two R89 radicals form together with the nitrogen atom to which they are bonded a 4 to 10-membered mono-, bi- or spirocyclic ring, which, apart from the nitrogen atom, comprises 0 to 3 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me; preferably H, (C₁-C₆)-alkyl, or the two R89 radicals form together with the nitrogen atom to which they are bonded a 5 to 6-membered monocyclic ring which, apart from the nitrogen atom, comprises 0 to 1 additional heteroatoms selected from the group of N, O and S and may additionally be substituted by one or more of the following substituents: F, Cl, Br, CF₃, O-(C₁-C₈)-alkyl, (C₁-C₆)-alkyl, CO(R71), oxo, OH, (C₁-C₄)-alkoxy-(C₁-C₄)-alkyl, hydroxy-(C₁-C₄)-alkyl, CON(R72)(R73), N(R74)CO(R75), N(R76)(R77), CO₂(R78), SO₂Me.

12. A compound as claimed in any of claims 1 to 11, wherein X is bond or N(R16), in which R16 is H or (C₁-C₈)-alkyl,

13. A medicament comprising one or more of the compounds of the formula I as claimed in one or more of claims 1 to 12.

14. A medicament comprising one or more of the compounds of the formula I as claimed in one or more of claims 1 to 12 and one or more active ingredients which have beneficial effects on metabolic disturbances or disorders associated therewith.

15. A medicament comprising one or more of the compounds of the formula I as claimed in one or more of claims 1 to 12 and one or more antidiabetics.

16. A medicament comprising one or more of the compounds of the formula I as claimed in one or more of claims 1 to 12 and one or more lipid modulators.

17. The use of the compounds of the formula I as claimed in one or more of claims 1 to 12 for the production of a medicament for the treatment and/or prevention of disorders of fatty acid metabolism and glucose utilization disorders.

18. The use of the compounds of the formula I as claimed in one or more of claims 1 to 12 for the production of a medicament for the treatment and/or prevention of disorders in which insulin resistance is involved.

19. The use of the compounds of the formula I as claimed in one or more of claims 1 to 12 for the production of a medicament for the treatment and/or prevention of diabetes mellitus and the sequelae associated therewith.

20. The use of the compounds of the formula I as claimed in one or more of claims 1 to 12 for the production of a medicament for the treatment and/or prevention of dyslipidemias and the sequelae thereof.

21. The use of the compounds of the formula I as claimed in one or more of claims 1 to 12 for the production of a medicament for the treatment and/or prevention of conditions associated with the metabolic syndrome.

22. The use of the compounds of the formula I as claimed in one or more of claims 1 to 12 for the production of a medicament for the treatment and/or prevention of obesity and sequelae associated therewith.

23. The use of the compounds as claimed in one or more of claims 1 to 12 in combination with at least one further active ingredient for the production of a medicament for the treatment and/or prevention of disorders of fatty acid metabolism and glucose utilization disorders.

24. The use of the compounds as claimed in one or more of claims 1 to 12 in combination with at least one further active ingredient for the production of a medicament for the treatment and/or prevention of disorders in which insulin resistance is involved.

25. A process for producing a medicament comprising one or more of the compounds as claimed in one or more of claims 1 to 12, which comprises mixing the active ingredient with a pharmaceutically suitable carrier, and converting this mixture into a form suitable for administration.

## Revendications

1. Composés de formule I 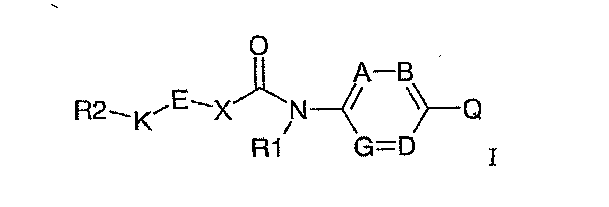 dans laquelle les symboles ont les significations suivantes
A, B, D, G indépendamment les uns des autres, N, C(R3), ou les groupes A et B ou D et G représentent chacun C(R3) et forment ensemble une unité ortho-phénylène, de manière à obtenir dans l'ensemble un système naphtalénique 1,4-disubstitué ; de préférence, indépendamment les uns des autres, N ou C(R3), le nombre total d'atomes d'azote dans le cycle étant compris entre 0 et 2, étant de préférence 0 ou 1 ; plus préférablement C(R3) ;
R3 H, F, CI, Br, CF₃, CN, O-(C₁-C₆)-alkyle, O-(C₁-C₄)-alcoxy-(C₁-C₄)-alkyle, S-(C₁-C₆)-alkyle, (C₁-C₆)-alkyle, (C₀-C₈)-alkylène-aryle, O-(C₀-C₈)-alkylène-aryle, N(R4)(R5), SO₂-CH₃, CON(R6)(R7), N(R8)CO(R9), CO(R12), (CR13R14)ₓ-O(R15) ; de préférence H, F, CI, Br, CF₃, CN, O-(C₁-C₆)-alkyle, (C₁-C₆)-alkyle, SO₂-CH₃, CON(R6)(R7), N(R8)CO(R9), CO(R12), (CR13R14)ₓ-O(R15); plus préférablement H, F, CI, CF₃, CN, (C₁-C₆)-alkyle, (C(R13)(R14))ₓ-O(R15) ; encore plus préférablement H, F, CI, (C₁-C₆)-alkyle ;
R4, R5, R6, R7, R8 indépendamment les uns des autres, H, (C₁-C₈)-alkyle ;
ou
R4 et R5, R6 et R7 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R9, R12 indépendamment les uns des autres, H, (C₁-C₈)-alkyle ;
R13, R14 H ;
R15 H, (C₁-C₆)-alkyle ;
x 0, 1, 2, de préférence 0, 1, plus préférablement 1 ;
R1 H, (C₁-C₈)-alkyle ;
X N(R16), une liaison, (R17)C=C(R18), C=C, CH₂-CH₂, YCH₂, CH₂Y, de préférence N(R16), une liaison ;
Y O, S, N(R21) ;
R16, R17, R18 indépendamment les uns des autres, H, (C₁-C₈)-alkyle ; de préférence H ;
R21 H, (C₁-C₈)-alkyle ;
E structure cyclique carbo- ou hétérocyclique bivalente ayant de 3 à 8 chaînons comportant de 0 à 4 hétéroatomes choisis dans le groupe constitué de N, O et S, pouvant éventuellement avoir des substituants choisis dans le groupe constitué de H, F, CI, Br, OH, CF₃, NO₂, CN, OCF₃, O-(C₁-C₆)-alkyle, O-(C₁-C₄)-alcoxy-(C₁-C₄)-alkyle, S-(C₁-C₆)-alkyle, (C₁-C₆)-alkyle, (C₂-C₆)-alcényle, O-(C₃-C₈)-cycloalkyle, (C₃-C₈)-cycloalcényle, (C₂-C₆)-alcynyle, (C₀-C₈)-alkylène-aryle, O-(C₀-C₈)-alkylène-aryle, S-aryle, N(R22)(R23), SO₂-CH₃, N(R26)CO(R27), N(R28)SO₂(R29), CO(R30) et être mono- ou bicyclique ; de préférence une structure cyclique carbo- ou hétérocyclique bivalente ayant de 5 à 7 chaînons comportant de 0 à 3 hétéroatomes choisis dans le groupe constitué de N, O et S, pouvant éventuellement avoir des substituants choisis dans le groupe constitué de H, F, CI, Br, OH, CF₃, NO₂, CN, OCF₃, O-(C₁-C₆)-alkyle, S-(C₁-C₆)-alkyle, (C₁-C₆)-alkyle, (C₂-C₆)-alcényle, O-(C₀-C₈)-alkylène-aryle, S-aryle, N(R22)(R23), SO₂-CH₃, N(R26)CO(R27), CO(R30) et être mono- ou bicyclique ; plus préférablement une structure cyclique carbo- ou hétérocyclique bivalente ayant de 5 à 7 chaînons comportant de 0 à 2 hétéroatomes choisis dans le groupe constitué de N, O et S, pouvant éventuellement avoir des substituants choisis dans le groupe constitué de H, F, CI, Br, OH, CF₃, NO₂, OCF₃, O-(C₁-C₆)-alkyle, (C₁-C₆)-alkyle, (C₂-C₆)-alcényle, N(R22)(R23), SO₂-CH₃, CO(R30), de préférence H, F, CI, Br, OH, CF₃, (C₁-C₆)-alkyle, O-(C₁-C₆)-alkyle, E est par exemple choisi dans le groupe constitué de 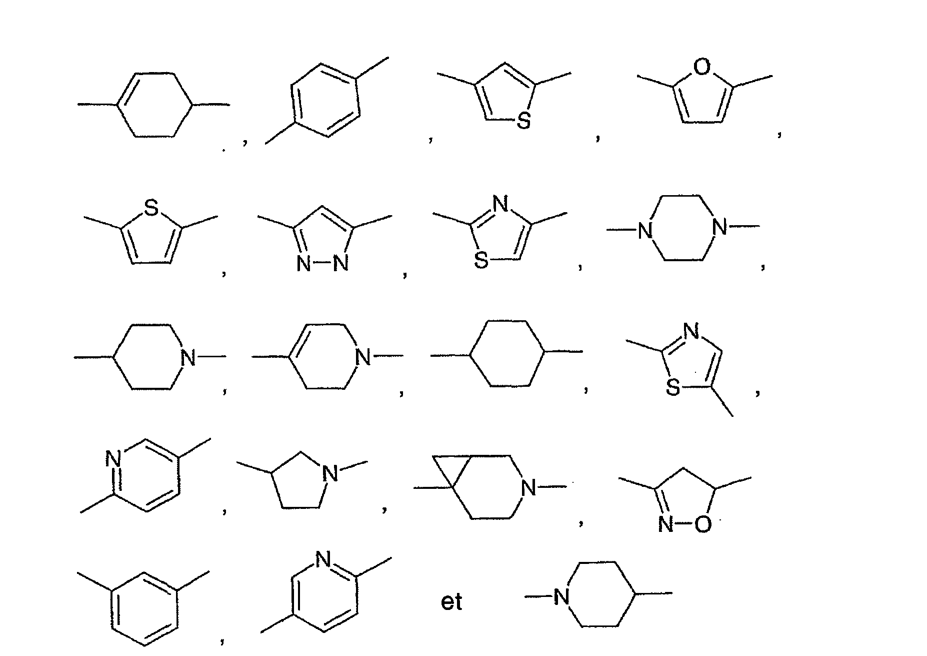 pouvant éventuellement avoir des substituants choisis dans le groupe constitué de H, F, CI, Br, OH, CF₃, NO₂, OCF₃, O-(C₁-C₆)-alkyle, (C₁-C₆)-alkyle, (C₂-C₆)-alcényle, N(R22)(R23), SO₂-CH₃, CO(R30), de préférence H, F, CI, Br, OH, CF₃, (C₁-C₆)-alkyle, O-(C₁-C₆)-alkyle ; de préférence 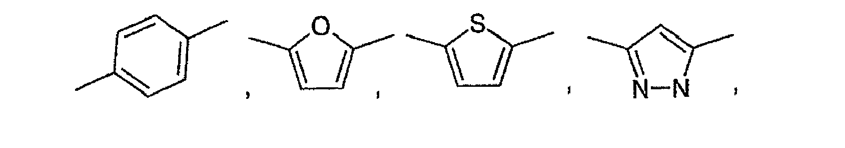 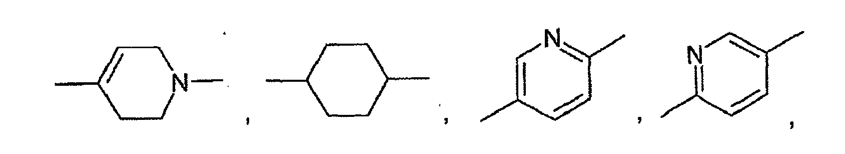 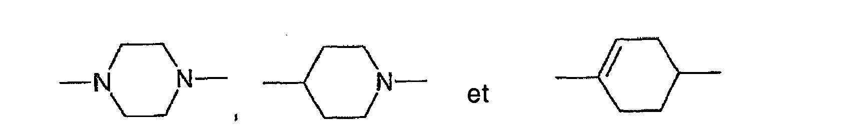 pouvant éventuellement avoir les substituants susmentionnés ;
R22, R23, R24, R25, R26, R28 indépendamment les uns des autres, H, (C₁-C₈)-alkyle ;
ou
R22 et R23, R24 et R25 indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R27,R29,R30 indépendamment les uns des autres, H, (C₁-C₈)-alkyle ;
K une liaison, O, OCH₂, CH₂O, S, SO, SO₂, N(R35), N(R36)CO, CON(R37), (C(R38)(R39))ᵥ, CO, (R31)C=C(R32), C=C, SCH₂, SO₂CH₂, plus préférablement OCH₂, CH₂O, N(R36)CO, CON(R37), (C(R38)(R39))₂, (R31)C=C(R32), C=C, SCH₂, SO₂CH₂, encore plus préférablement OCH₂, CH₂O, CON(R37), C=C, SCH₂ ;
v 2;
R31, R32, R35, R36, R37, R38, R39 indépendamment les uns des autres, H, (C₁-C₈)-alkyle ;
R2 (C₁-C₈)-alkyle, (C₁-C₄)-alcoxy-(C₁-C₄)-alkyle, un cycle mono- , bi-, tri- ou spirocyclique ayant de 3 à 10 chaînons pouvant comprendre de 0 à 3 hétéroatomes choisis dans le groupe constitué de l'oxygène, l'azote et le soufre, le système cyclique pouvant additionnellement être substitué par un ou plusieurs des substituants suivants : F, CI, Br, CF₃, CN, (C₁-C₆)-alkyle, O-(C₁-C₈)-alkyle, (C₀-C₂)-alkylène-aryle, oxo, CO(R41), CON(R42)(R43), hydroxy, N(R45)CO(C₁-C₆)-alkyle, N(R46)(R47) ou SO₂CH₃ ; de préférence (C₁-C₈)-alkyle, (C₁-C₄)-alcoxy-(C₁-C₄)-alkyle, un cycle mono- ou bicyclique ayant de 3 à 10 chaînons pouvant comprendre de 0 à 2 hétéroatomes choisis dans le groupe constitué par l'oxygène, l'azote et le soufre, le système cyclique pouvant additionnellement être substitué par un ou plusieurs des substituants suivants : F, CI, Br, CF₃, CN, (C₁-C₆)-alkyle, O-(C₁-C₈)-alkyle, oxo, CO(R41), CON(R42)(R43), N(R45)CO(C₁-C₆)-alkyle ou SO₂CH₃ ;
R41, R42, R43, R45, R46, R47 indépendamment les uns des autres, H, (C₁-C₈)-alkyle ;
ou
R42 et R43, R46 et R47 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
Q structure cyclique bi-, tri- ou spirocyclique, saturée ou partiellement insaturée, comportant un atome d'azote et de 0 à 3 hétéroatomes supplémentaires choisis dans le groupe constitué par N, O et S, les cycles de la structure pouvant être spiro-liés, condensés ou pontés, et le système cyclique pouvant être substitué par un ou plusieurs des substituants suivants : F, OH, CF₃, CN, OCF₃, oxo, O-(C₁-C₈)-alkyle, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)ₚ-R55 ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)-alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO₂(R62), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R63), oxo, OH ;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70 ; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, Cl, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ;
R64, R65, R66, R68, R69, R71,
R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, Cl, Br, CF₃, (C₁-C₈)-alkyle, O-(C₁-C₈)-alkyle, CO(R86), oxo, OH ;
R79, R80, R81, R82, R83, R84, R85, R86 indépendamment les uns des autres, H, (C₁-C₈)-alkyle
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ; les N-oxydes de ces composés et les sels physiologiquement tolérables de ces composés.

2. Composés selon la revendication 1, dans lesquels Q représente : 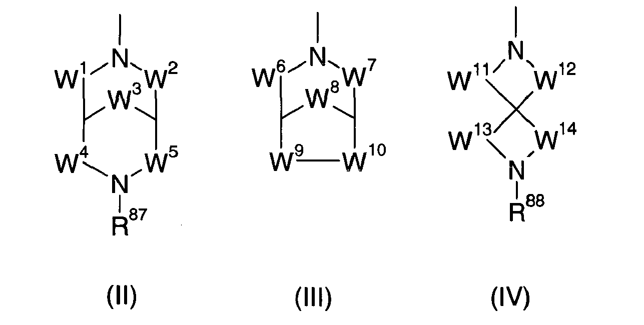 les symboles ayant les significations suivantes
W1, W2, W3, W4, W5, W6, W7, W8,
W9, W10, W11, W12, W13, W14 indépendamment les uns des autres, une liaison, C=C, une chaîne alkylène ou alkylidène ayant de 1 à 4 éléments dans laquelle de 0 à 1 des atomes de carbone, mis à part une double liaison présente dans la chaîne alkylidène, peut être remplacé par un élément choisi dans le groupe constitué de N(R90), O et S ;
les atomes de carbone dans les groupes de formules (II), (III) et (IV) pouvant être substitués par H, F, OH, oxo, (C₁-C₆)-alkyle, O-(C₁-C₆)-alkyle, (CR52R53)ₒR54, de préférence H, (CR52R53)ₒR54 ;
R87, R88, R90 H, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)ₚ-R55 ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)-alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO₂(R62), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, Cl, Br, CF₃, (C₁-C₈)-alkyle, O-(C₁-C₈)-alkyle, CO(R63), oxo, OH;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisis dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70 ; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ;
R64, R65, R66, R68, R69, R71,
R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85),SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R86), oxo, OH;
R79, R80, R81, R82, R83, R84, R85, R86 indépendamment les uns des autres, H, (C₁-C₈)-alkyle ;
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ; les N-oxydes de ces composés et les sels physiologiquement tolérables de ces composés.

3. Composés selon la revendication 2, dans lesquels Q représente : 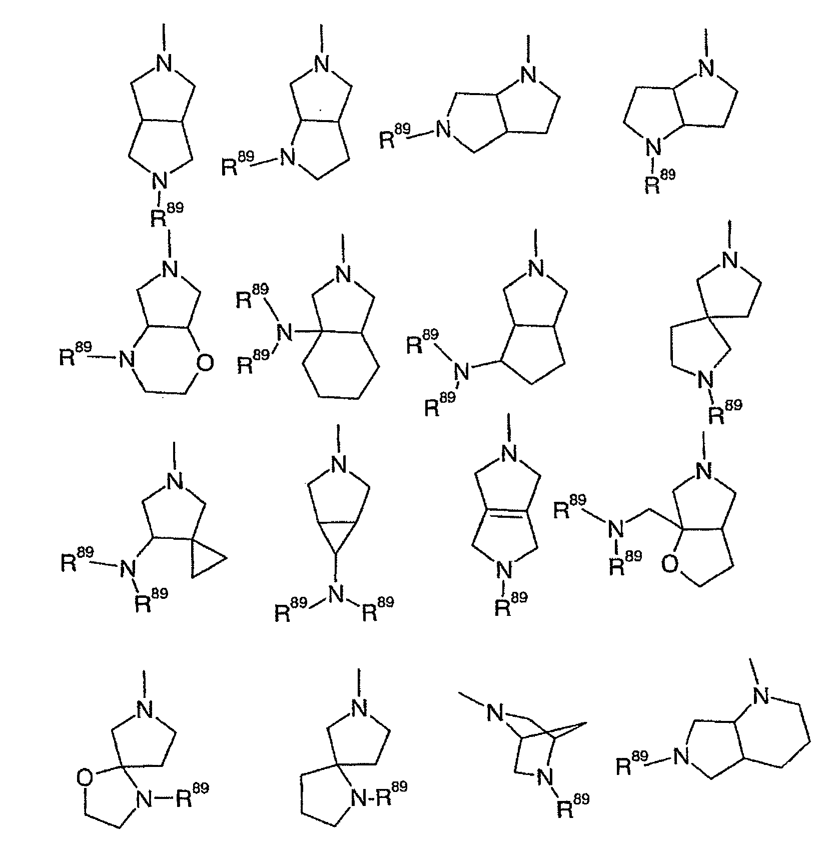 les symboles ayant les significations suivantes : R89 dans le groupe N-R89 :
H, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)ₚ-R55 ; de préférence H, (C₁-C₆)-alkyle ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)-alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO2(R62), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R63), oxo, OH ;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, Cl, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle,CON(R72)(R73), N(R74)CO(R75), N(R76)(R77), CO₂(R78), SO₂Me ;
R64, R65, R66, R68, R69,
R71, R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85), SO₂Me, CN, un système cyclique ayant de 3 à 10 chaînons comportant de 0 à 3 hétéroatomes choisis dans le groupe constitué de N, O et S, pouvant être substitué par F, CI, Br, CF₃, (C₁-C₈)-alkyle, O-(C₁-C₈)-alkyle, CO(R86), oxo, OH ;
R79, R80, R81, R82, R83, R84, R85, R86 indépendamment les uns des autres, H, (C₁-C₈)-alkyle ;
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R89 dans le groupe N(R89)₂ : indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70; ou les deux radicaux R89 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ; de préférence H, (C₁-C₆)-alkyle, ou les deux radicaux R89 forment avec l'atome d'azote auquel ils sont liés un cycle monocyclique ayant de 5 à 6 chaînons comprenant, outre l'atome d'azote, de 0 à 1 hétéroatome supplémentaire choisi dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me.

4. Composés de formule I selon l'une quelconque des revendications 1
à 3, **caractérisés en ce que**
A, B, D, G sont, indépendamment les uns des autres, N ou C(R3), le nombre total d'atomes d'azote dans ce cycle étant compris entre 0 et 2, de préférence 0 ou 1, plus préférablement 0.

5. Composés de formule I selon l'une quelconque des revendications 1 à 4,
dans lesquels les symboles ont les significations suivantes
K liaison, O, CO, OCH₂, CH₂O, N(R36)CO, CON(R37), (C(R38)(R39))₂, (R31)C=C(R32), C≡C, SCH₂, SO₂CH₂, plus préférablement OCH₂, CH₂O, N(R36)CO, CON(R37), (C(R38)(R39))₂, (R31)C=C(R32), C=C, SCH₂, SO₂CH₂, encore plus préférablement OCH₂, CH₂O, CON(R37), (C(R38)(R39))₂, C=C, SCH₂ ;
R31, R32, R36, R37, R38, R39 étant, indépendamment les uns des autres, H, (C₁-C₈)-alkyle.

6. Composés selon l'une quelconque des revendications 1 à 5, dans lesquels les symboles ont les significations suivantes
R2 (C₁-C₈)-alkyle, (C₁-C₄)-alcoxy-(C₁-C₄)-alkyle, un cycle mono- , bi-, tri- ou spirocyclique ayant de 3 à 10 chaînons pouvant comprendre de 0 à 3 hétéroatomes choisis dans le groupe constitué de l'oxygène, l'azote et le soufre, le système cyclique pouvant additionnellement être substitué par un ou plusieurs des substituants suivants : F, Cl, Br, CF₃, CN, (C₁-C₆)-alkyle, O-(C₁-C₈)-alkyle, (C₀-C₂)-alkylène-aryle, oxo, CO(R41), CON(R42)(R43), hydroxy, N(R45)CO(C₁-C₆)-alkyle, N(R46)(R47) ou SO₂CH₃ ; de préférence (C₁-C₈)-alkyle, (C₁-C₄)-alcoxy-(C₁-C₄)-alkyle, un cycle mono- ou bicyclique ayant de 3 à 10 chaînons pouvant comprendre de 0 à 2 hétéroatomes choisis dans le groupe constitué par l'oxygène, l'azote et le soufre, le système cyclique pouvant additionnellement être substitué par un ou plusieurs des substituants suivants : F, CI, Br, CF₃, CN, (C₁-C₆)-alkyle, O-(C₁-C₈)-alkyle, oxo, CO(R41), CON(R42)(R43), N(R45)CO(C₁-C₆)-alkyle ou SO₂CH₃ ;
R41, R42, R43, R45, R46, R47 indépendamment les uns des autres, H, (C₁-C₈)-alkyle ;
ou
R42 et R43, R46 et R47 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre.

7. Composés de formule I selon l'une quelconque des revendications 1 à 6,
dans lesquels les symboles ont les significations suivantes
A, B, D, G C(R3) ;
R3 H, F, CI, Br, CF₃, CN, O-(C₁-C₆)-alkyle, O-(C₁-C₄)-alcoxy-(C₁-C₄)-alkyle, S-(C₁-C₆)-alkyle, (C₁-C₆)-alkyle, (C₀-C₈)-alkylène-aryle, O-(C₀-C₈)-alkylène-aryle, N(R4)(R5), SO₂-CH₃, CON(R6)(R7), N(R8)CO(R9), CO(R12), (CR13R14)ₓ O(R15) ; de préférence H, F, CI, Br, CF₃, CN, O-(C₁-C₆)-alkyle, (C₁-C₆)-alkyle, SO₂-CH₃, CON(R6)(R7), N(R8)CO(R9), CO(R12), (CR13R14)ₓ-O(R15); plus préférablement H, F, CI, CF₃, CN, (C₁-C₆)-alkyle, (C(R13)(R14))ₓ-O(R15); encore plus préférablement H, F, CI, (C₁-C₆)-alkyle ; encore mieux H, CH₃, F ;
R4, R5, R6, R7, R8 indépendamment les uns des autres, H, (C₁-C₈)-alkyle ;
ou
R4 et R5, R6 et R7 indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R9, R12 indépendamment les uns des autres, H, (C₁-C₈)-alkyle ;
R13, R14 H;
R15 H, (C₁-C₆)-alkyle ;
x 0, 1, 2, de préférence 0, 1, plus préférablement 1.

8. Composés selon la revendication 1,
dans lesquels les symboles ont les significations suivantes
Q structure cyclique bi-, tri- ou spirocyclique, saturée ou partiellement insaturée, comportant un atome d'azote et de 0 à 3 hétéroatomes supplémentaires choisis dans le groupe constitué par N, O et S, les cycles de la structure pouvant être spiro-liés, condensés ou pontés, et le système cyclique pouvant être substitué par un ou plusieurs des substituants suivants : F, OH, CF₃, CN, OCF₃, O-(C₁-C₈)-alkyle, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)ₚ-R55 ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)-alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO2(R62), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R63), OH ;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70 ; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ;
R64, R65, R66, R68, R69,
R71, R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85), SO₂Me, CN, un système cyclique ayant de 3 à 10 chaînons comportant de 0 à 3 hétéroatomes choisis dans le groupe constitué de N, O et S, pouvant être substitué par F, CI, Br, CF₃, (C₁-C₈)-alkyle, O-(C₁-C₈)-alkyle, CO(R86), oxo, OH ;
R79, R80, R81, R82, R83, R84, R85, R86 indépendamment les uns des autres, H, (C₁-C₈)-alkyle ;
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre.

9. Composés selon la revendication 1,
dans lesquels les symboles ont les significations suivantes
Q une structure cyclique bi-, tri- ou spirocyclique saturée comportant un atome d'azote et de 0 à 3 hétéroatomes supplémentaires choisis dans le groupe constitué par N, O et S, les cycles de la structure pouvant être spiro-liés, condensés ou pontés, et le système cyclique pouvant être substitué par un ou plusieurs des substituants suivants : F, OH, CF₃, CN, OCF₃, oxo, O-(C₁-C₈)-alkyle, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)p-R55 ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO₂(R62), SO₂Me, CN, un système cyclique ayant de 3 à 10 chaînons comportant de 0 à 3 hétéroatomes choisis dans le groupe constitué de N, O et S, pouvant être substitué par F, CI, Br, CF₃, (C₁-C₈)-alkyle, O-(C₁-C₈)-alkyle, CO(R63), oxo, OH ;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70 ; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ;
R64, R65, R66, R68, R69, R71,
R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85),SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R86), oxo, OH ;
R79, R80, R81, R82, R83, R84, R85, R86 indépendamment les uns des autres, H, (C₁-C₈)-alkyle ;
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre.

10. Composés selon l'une quelconque des revendications 2 à 9, **caractérisés en ce que** W3 dans la formule II et W8 dans la formule III sont chacun une liaison.

11. Composés selon la revendication 9, dans lesquels Q représente : 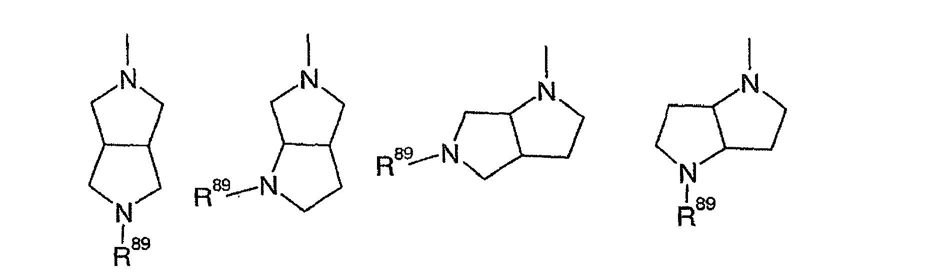 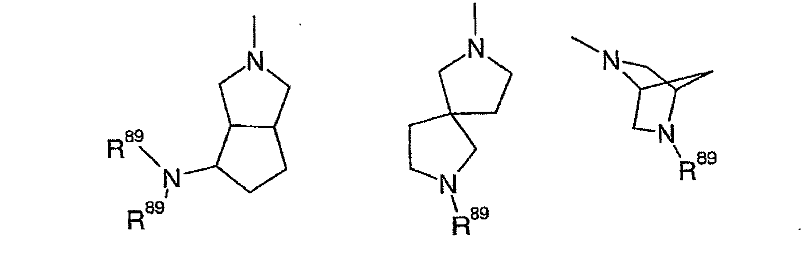 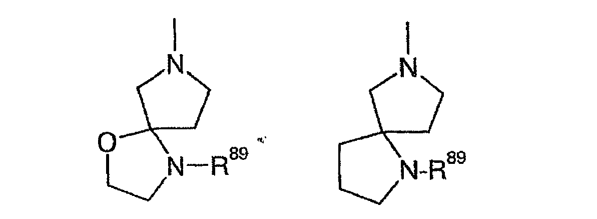 les symboles ayant les significations suivantes :
R89 dans le groupe N-R89 : H, (C₁-C₄)-alcoxy-(C₁-C₄)-alkyle, (C₁-C₆)-alkyle, (C₂-C₆)-alcényle, (C₂-C₆)-alcynyle, CO(R51), (CR52R53)ₒ-R54, CO(CR52R53)ₚ-R55 ; de préférence H, (C₁-C₆)-alkyle ;
R51 H, (C₁-C₈)-alkyle ;
R52, R53 indépendamment l'un de l'autre, H, (C₁-C₈)-alkyle, OH, (C₃-C₈)-cycloalkyle, (C₁-C₄)-alcoxy-(C₁-C₄)-alkyle ;
o, p indépendamment l'un de l'autre, 0, 1, 2, 3, 4, 5, 6 ;
R54, R55 indépendamment l'un de l'autre, OH, O-(C₁-C₈)-alkyle, CON(R56)(R57), N(R58)CO(R59), N(R60)(R61), CO₂(R62), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R63), oxo, OH ;
R56, R57, R58, R59, R62, R63 indépendamment les uns des autres, H, (C₁-C₈)-alkyle ;
ou
R56 et R57 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R60, R61 indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70; ou R60 et R61 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ;
R64, R65, R66, R68, R69, R71,
R72, R73, R74, R75, R76, R77, R78 indépendamment les uns des autres, H, (C₁-C₈)-alkyle ;
ou
R76 et R77 forment, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
q, r indépendamment l'un de l'autre, 0, 1, 2, 3, 4, 5, 6 ;
R67, R70 indépendamment l'un de l'autre, OH, O-(C₁-C₈)-alkyle, CON(R79)(R80), N(R81)CO(R82), N(R83)(R84), CO₂(R85), SO₂Me, CN, un système cyclique ayant de 3 à 10 chaînons comportant de 0 à 3 hétéroatomes choisis dans le groupe constitué de N, O et S, pouvant être substitué par un ou plusieurs des substituants suivants : F, CI, Br, CF₃, (C₁-C₈)-alkyle, O-(C₁-C₈)-alkyle, CO(R86), oxo, OH ;
R79, R80, R81, R82, R83, R84, R85, R86 indépendamment les uns des autres, H, (C₁-C₈)-alkyle ;
ou
R79 et R80, R83 et R84 forment, indépendamment les uns des autres, éventuellement avec l'atome d'azote auquel ils sont liés, un cycle à 5 ou 6 chaînons, qui, outre l'atome d'azote, peut également comprendre de 0 à 1 hétéroatome supplémentaire choisi dans le groupe constitué de NH, N-(C₁-C₆)-alkyle, oxygène et soufre ;
R89 dans le groupe N(R89)₂ : indépendamment l'un de l'autre, H, (C₁-C₆)-alkyle, (C₁-C₄)-alcoxy-(C₁-C₄)-alkyle, (C₂-C₆)-alcényle, (C₂-C₆)-alcynyle, CO(R64), (CR65R66)q-R67, CO(CR68R69)ᵣ-R70; ou les deux radicaux R89 forment avec l'atome d'azote auquel ils sont liés un cycle mono-, bi- ou spirocyclique ayant de 4 à 10 chaînons comprenant, outre l'atome d'azote, de 0 à 3 hétéroatomes supplémentaires choisis dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me ; de préférence H, (C₁-C₆)-alkyle, ou les deux radicaux R89 forment avec l'atome d'azote auquel ils sont liés un cycle monocyclique ayant de 5 à 6 chaînons comprenant, outre l'atome d'azote, de 0 à 1 hétéroatome supplémentaire choisi dans le groupe constitué de N, O et S, et pouvant additionnellement être substitué par un ou plusieurs des substituants suivants : F, CI, Br, CF₃, O-(C₁-C₈)-alkyle, (C₁-C₆)-alkyle, CO(R71), oxo, OH, (C₁-C₄)-alcoxy-(C₁-C₄)-alkyle, hydroxy-(C₁-C₄)-alkyle, CON(R72)(R73), N(R74)CO(R75), N(R76)(R77), CO₂(R78), SO₂Me.

12. Composés selon l'une quelconque des revendications 1 à 11, **caractérisés en ce que** X est une liaison ou N(R16), R16 étant H ou (C₁-C₈)-alkyle.

13. Médicament comprenant un ou plusieurs des composés de formule I selon une ou plusieurs des revendications 1 à 12.

14. Médicament comprenant un ou plusieurs des composés de formule I selon une ou plusieurs des revendications 1 à 12, ainsi qu'un ou plusieurs ingrédients actifs ayant des effets bénéfiques sur des perturbations métaboliques ou les troubles leur étant associés.

15. Médicament comprenant un ou plusieurs des composés de formule I selon une ou plusieurs des revendications 1 à 12, ainsi qu'un ou plusieurs antidiabétiques.

16. Médicament comprenant un ou plusieurs des composés de formule I selon une ou plusieurs des revendications 1 à 12, ainsi qu'un ou plusieurs modulateurs lipidiques.

17. Utilisation des composés de formule I selon une ou plusieurs des revendications 1 à 12 pour la fabrication d'un médicament destiné au traitement et/ou à la prévention de troubles du métabolisme des acides gras et de troubles de l'utilisation du glucose.

18. Utilisation des composés de formule I selon une ou plusieurs des revendications 1 à 12 pour la fabrication d'un médicament destiné au traitement et/ou à la prévention de troubles impliquant une résistance à l'insuline.

19. Utilisation des composés de formule I selon une ou plusieurs des revendications 1 à 12 pour la fabrication d'un médicament destiné au traitement et/ou à la prévention du diabète sucré et des séquelles associées.

20. Utilisation des composés de formule I selon une ou plusieurs des revendications 1 à 12 pour la fabrication d'un médicament destiné au traitement et/ou à la prévention des dyslipidémies et des séquelles associées.

21. Utilisation des composés de formule I selon une ou plusieurs des revendications 1 à 12 pour la fabrication d'un médicament destiné au traitement et/ou à la prévention des maladies associées au syndrome métabolique.

22. Utilisation des composés de formule I selon une ou plusieurs des revendications 1 à 12 pour la fabrication d'un médicament destiné au traitement et/ou à la prévention de l'obésité et des séquelles associées.

23. Utilisation des composés selon une ou plusieurs des revendications 1 à 12 en combinaison avec au moins un ingrédient actif supplémentaire pour la fabrication d'un médicament destiné au traitement et/ou à la prévention de troubles du métabolisme des acides gras et de troubles de l'utilisation du glucose.

24. Utilisation des composés selon une ou plusieurs des revendications 1 à 12 en combinaison avec au moins un ingrédient actif supplémentaire pour la fabrication d'un médicament destiné au traitement et/ou à la prévention de troubles impliquant une résistance à l'insuline.

25. Procédé de fabrication d'un médicament comprenant un ou plusieurs des composés selon une ou plusieurs des revendications 1 à 12, **caractérisé par** le mélange de l'ingrédient actif avec un véhicule pharmaceutiquement approprié, et la transformation de ce mélange en une forme convenable pour l'administration.
